# EUROPÄISCHE PATENTSCHRIFT

(11) **EP 1 366 008 B1**
(45) Veröffentlichungstag und Bekanntmachung des Hinweises auf die Patenterteilung: **22.11.2006**
(21) Anmeldenummer: 02719815.9
(22) Anmeldetag: 13.02.2002
(51) Int. Cl.: C07C 45/40, C07C 67/00, C07C 69/716, C07C 69/67, C07C 67/313, C07C 67/333, C07C 67/31, C07C 69/68, C07D 213/48, C07C 67/293

(54) **VERFAHREN ZUR HERSTELLUNG VON MONO- ODER BISCARBONYL- ODER HYDROXYLVERBINDUNGEN**
METHOD FOR PRODUCING MONOCARBONYL COMPOUNDS OR BISCARBONYL COMPOUNDS OR HYDROXYL COMPOUNDS
PROCEDE DE FABRICATION DE COMPOSES MONOCARBONYL OU BISCARBONYL OU HYDROXYL

(30) Priorität: 09.03.2001 AT 3702001
(43) Veröffentlichungstag der Anmeldung: 03.12.2003
(73) Patentinhaber: DSM Fine Chemicals Austria Nfg GmbH & Co KG, 4021 Linz (AT)
(72) Erfinder: KLOIMSTEIN, Engelbert, A-4070 Eferding (AT); SAJTOS, Alexander, A-4060 Leonding (AT); ZIMMERMANN, Curt, A-4312 Rie in der Riedmark (AT)
(74) Vertreter: Lindinger, Ingrid
(86) Internationale Anmeldenummer: PCT/EP2002/001478
(87) Internationale Veröffentlichungsnummer: WO 2002/072518

(56) Entgegenhaltungen:
- EP-A- 1 095 700
- US-A- 4 242 309
- PATENT ABSTRACTS OF JAPAN vol. 017, no. 513 (C-1111), 16. September 1993 (1993-09-16) & JP 05 140030 A (AGENCY OF IND SCIENCE & TECHNOL;OTHERS: 01), 8. Juni 1993 (1993-06-08)

## Beschreibung

Die Erfindung betrifft ein Verfahren zur Herstellung von Mono- oder Biscarbonyl-oder Hydroxylverbindungen aus ungesättigten organischen Kohlenstoffverbindungen mit einer oder mehreren olefinischen oder aromatischen Doppelbindungen im Molekül.

Die Ozonolyse von Olefinen liefert, auf umweltfreundliche Weise Carbonylverbindungen, wie Aldehyde oder Ketone, oder, je nach den Aufarbeitungsbedingungen, deren Halbacetale, Acetale oder Ketale, sowie Hydroxylverbindungen, die wertvolle Ausgangsstoffe in der präparativen, organischen Chemie darstellen.

Die Herstellung von Carbonyl- oder Hydroxylverbindungen aus organischen Verbindungen, die als Strukturelement eine oder mehrere C = C-Doppelbindungen im Molekül aufweisen, mittels eines zweistufigen Ozonolyse- und Reduktionsprozesses ist bekannt. Bei der Durchführung dieser Methode wird in der ersten Stufe zur Erzielung einer möglichst vollständigen Ozonisierung der Doppelbindung meistens mit einem Ozonüberschuß gearbeitet. Die in der zweiten Stufe folgende reduktive Spaltung bereitet immer wieder Schwierigkeiten, da die peroxidhältigen Ozonisierungsprodukte instabil sind und in Abwesenheit von metallischen Hydrierkatalysatoren besonders leicht Umlagerungen oder Zersetzung erfahren, bevor sie zu den entsprechenden Carbonylverbindungen reduziert werden können. Außerdem werden bei Edelmetallkatalysatoren bei längerem Kontakt mit peroxidhältigen Lösungen Aktivitätsverluste des Katalysators beobachtet, sodass die Lösungen bei der reduktiven Spaltung durch Hydrierung in der Regel nicht gänzlich peroxidfrei werden und neben Schwierigkeiten bei der Reindarstellung der Endprodukte auch Ausbeuteverluste und eine Explosionsgefahr hingenommen werden müssen.

Zur Vermeidung dieser Schwierigkeiten wird in US-PS-3 145 232 ein Verfahren zur Herstellung von Carbonylverbindungen empfohlen, bei der die reduktive Spaltung nach der Ozonolyse bei Temperaturen unterhalb von -40°C in Anwesenheit eines Trialkylphosphits durchgeführt wird. Neben dem apparativen Aufwand zur Herstellung der extrem tiefen Reaktionstemperaturen erfordert eine solche Reaktionsführung die Verwendung von absolut wasserfreien Lösungsmitteln, da die Trialkylphosphite in wasserhaltigen Lösungsmitteln äußerst rasch hydrolysiert werden. Außerdem bereitet die Abtrennung der freien Carbonylverbindungen von den bei der Reduktion entstehenden Phosphatestern erhebliche Schwierigkeiten.

Da nachgewiesen wurde, dass sich tiefe Reaktionstemperaturen nachteilig auf die Aktivität der eingesetzten Reduktionsmittel auswirken und deshalb Ausbeuteverluste entstehen, wird nach einem Verfahren zur Herstellung von aliphatischen, aromatischen und heteroaromatischen Aldehyden, wie es in US-PS-3 637 721 beschrieben ist, zwar die Ozonolyse der C=C-Doppelbindung bei -50°C durchgeführt, während die Reaktionstemperaturen im Verlaufe der reduktiven Spaltung der Ozonisierungsprodukte mit aromatischen oder aliphatischen Disulfiden bis auf 50°C gesteigert wird. Beim genannten Verfahren gestaltet sich aber die Abtrennung der bei der Reduktion als Begleitprodukte entstehenden Sulfoxide, beispielsweise Dimethylsulfoxid, von den als Verfahrensprodukte entstehenden Aldehyden als äußerst schwierig und ist in vielen Fällen ohne Derivatisierung der Aldehyde überhaupt undurchführbar.

In US-PS-3 705 922 oder DE-OS-2 514 001 ist schließlich die Herstellung von Carbonylverbindungen mittels eines Ozonolyse- und Reduktionsverfahrens beschrieben, bei dem die als Ausgangsmaterial dienenden ungesättigten Verbindungen mit einem Überschuss an Ozon umgesetzt und die dabei gebildeten Ozonisierungsprodukte durch katalytische Hydrierung reduktiv gespalten werden. Dabei muss überschüssiges Ozon aber vor der reduktiven Aufspaltung zum Schutz des Hydrierkatalysators vor Aktivitätsverlusten durch Spülung der Reaktionslösung mit einem Inertgas, beispielsweise mit Stickstoff, in einem eigenen Arbeitsgang wieder entfernt werden.

Zur Durchführung der Hydrierung setzt man dann dem bei der Ozonolyse gebildeten Reaktionsgemisch den Katalysator, der bevorzugt ein Edelmetallkatalysator ist, direkt zu und leitet bis zur Sättigung Wasserstoff ein.

Da Edelmetallkatalysatoren bei längerem Kontakt mit organischen Peroxiden deaktiviert werden, hängt bei den bekannten Verfahren die Ausbeute bei der Hydrierung von der Menge des jeweils eingesetzten Hydrierkatalysators ab. Wie aus einem Vergleich der Beispiele in US-PS-3 705 922 hervorgeht, nimmt die Ausbeute trotz entsprechend verlängerter Reaktionszeit um etwa 10 % ab, wenn bei gleicher Ansatzgröße anstelle von 0.5 g nur 0.2 g eines Pd/Al₂O₃-Katalysators verwendet werden. In den genannten Druckschriften finden sich aber auch keine Angaben über die Möglichkeiten zur Regenerierung oder Wiederverwendung der eingesetzten Edelmetallkatalysatoren nach Beendigung der Hydrierung.

Verfahren zur Herstellung von Carbonylverbindungen, deren Halbacetalen, Acetalen oder Ketalen durch Ozonolyse und Reduktion, die obige Nachteile vermeiden sollen und die in technischem Maßstab durchgeführt werden, sind in EP-B-0 146 784 oder EP-B-0 147 593 beschrieben. Gemäß der Offenbarung dieser beiden Patentschriften werden Verbindungen, die olefinische Doppelbindungen aufweisen, in einem niedrigen aliphatischen Alkohol bei Temperaturen von -80 °C bis 20 °C mit der äquivalenten Menge Ozon umgesetzt, worauf die peroxidische Reaktionslösung in eine Suspension eines Hydrierkatalysators unter Zugabe von Wasserstoff in einer solchen Weise eingespeist wird, dass in der Reaktionsmischung eine Peroxidkonzentration von 0,1 Mol/l nicht überschritten wird. Da bei dieser Art der Reaktionsführung saure Nebenprodukte entstehen, die den Katalysator vergiften und rasch deaktivieren würden, muss der pH- Wert der Reaktionsmischung durch Zugabe einer Base kontrolliert werden.

Bei den bisher bekannten Verfahrensvarianten werden sowohl der Ozonolyseschritt als auch die Hydrierung batchweise durchgeführt. Auch der zumeist verwendete Ozonüberschuss wirkt sich bei diesen Verfahren negativ aus, da dieser beispielsweise vor dem Hydrierungsschritt mittels Inertgas ausgeblasen werden muss.

In DE 27 13 863 ist eine kontinuierliche Ozonolyse, insbesondere von langkettigen bzw. höher molekularen Verbindungen, wie Olefine, Ölsäure oder Linolsäure, in Gegenwart von Wasser beschrieben. Das Wasser wird dabei anstelle eines externen Kühlkreislauf verwendet und dient somit zur in situ Abführung der Reaktionswärme. Dieses Verfahren ist lediglich für schnellreagierende Substrate, wie Ölsäure und nur für wässrige Systeme, nicht aber für rein organische Systeme, die aber in der Mehrzahl in der Ozonolyse verwendet werden.

In US 4 242 309 ist ein Verfahren zur kontinuierlichen Ozonolyse von ungesättigten Verbindungen bei 10 bis 50°C in Gegenwart von Wasser beschrieben. Das Wasser dient dabei dazu, um die Reaktionswärme durch in situ Verdunstung abzuführen ohne externe Kühlung. Die Eduktströme werden dabei beide von oben in die jeweilige Reaktionsapparatur eingebracht.

Es wurde nun unerwarteterweise gefunden, dass die den bekannten Verfahren anhaftenden Nachteile gemäß der vorliegenden Erfindung durch ein einfaches und ökonomisches Verfahren vermieden werden können, bei dem man in einer kontinuierlichen Fahrweise eine ungesättigte organische Kohlenstoffverbindung mit einer oder mehreren olefinischen oder aromatischen Doppelbindungen, trotz bekannter Nachteile, mit einem Ozonüberschuss umsetzt und anschließend die peroxidhältigen Ozonisierungsprodukte, ebenfalls in einer kontinuierlichen Fahrweise, in verdünnter Lösung bei einer niedrigen Konzentration an Peroxiden durch katalytische Hydrierung rasch reduktiv spaltet oder mittels Oxidation oder einfachem Erwärmen in die gewünschten Endprodukte überführt.

Im Vergleich zu den bekannten Verfahren liefert das Verfahren nach der vorliegenden Erfindung durch die kontinuierliche Prozessführung auf einfacherem und wirtschaftlicherem Weg Carbonyl- oder Hydroxylverbindungen in vergleichbarer Ausbeute und Reinheit, wobei sich insbesondere die gleichbleibenden und einfach zu kontrollierenden Parameter, die im geringeren Ausmaß benötigte Überwachung und der geringere Peroxidgehalt in der Anlage als besonders vorteilhaft erweisen. Die Katalysatoren werden beim erfindungsgemäßen Verfahren geschont und in keiner Weise während einer längeren Betriebsdauer chemisch vergiftet, sodass sie erstens über Jahre stabil bleiben und zweitens auch ohne Regenerierung und Aufarbeitung bei der Wiederverwendung keinen merkbaren Aktivitätsverlust zeigen. Alle diese vorteilhaften Eigenschaften waren im Hinblick auf den Stand der Technik nicht zu erwarten.

Gegenstand der vorliegenden Erfindung ist demnach ein Verfahren zur Herstellung von Mono- oder Biscarbonyl- oder Hydroxylverbindungen durch Ozonisierung von ungesättigten organischen Kohlenstoffverbindungen, die eine oder mehrere durch Ozon spaltbare olefinische oder aromatische Doppelbindungen im Molekül aufweisen und anschließender Aufarbeitung der Ozonisierungsprodukte, das dadurch gekennzeichnet ist, dass ungesättigte organische Kohlenstoffverbindungen, die eine oder mehrere durch Ozon spaltbare olefinische oder aromatische Doppelbindungen im Molekül aufweisen,
a) in einem organischen Lösungsmittel oder in einer wässrigen Lösung in 1 bis 2 Schritten kontinuierlich in einer Vorrichtung bestehend aus zwei Absorptionsapparaturen, Vorrichtungen zur Abfuhr der Reaktionswärme und Vorrichtungen zum Trennen von Gas- und Flüssigphase, mit gegenläufigen Eduktströmen mit Ozon in stöchiometrischen Mengen oder im Überschuss umgesetzt und
b) die dabei entstehenden Peroxide in Abhängigkeit von den Reaktionsparametern aus Schritt a) entweder durch kontinuierliche oder diskontinuierliche Hydrierung, Oxidation oder Erwärmen in die entsprechenden Mono- oder Biscarbonyl- oder Hydroxylverbindungen überführt werden,
wobei in Schritt a)
das Edukt mit einer vom eingesetzten Edukt und den Reaktionsbedingungen abhängigen Ausgangskonzentration in die erste Absorptionsapparatur eingespeist, der Ozon-führende O₂-Strom mit einer von der Reaktivität des Eduktes abhängigen Ozonkonzentration hingegen in die zweiten Absorptionsapparatur eingebracht wird, sodass in der 1. Absorptionsapparatur das eingesetzte Edukt mit dem Ozonstrom in Kontakt gebracht wird, der nach Durchlauf der 2. Absorptionsapparatur in die 1. Absorp-tionsapparatur eingespeist wird, wodurch in der 1. Absorptionsapparatur ein Ozonunterschuss vorliegt,
anschließend das Reaktionsgemisch, nach der Reaktion des in die 1. Absorptionsapparatur eingespeisten Ozons mit dem entsprechend eingebrachten Edukts, aus der 1. Absorptionsapparatur austritt, in eine Gasphase und eine flüssige Phase getrennt wird, worauf die flüssige Phase, die noch nicht umgesetztes Edukt, Lösungsmittel und das entsprechende Ozonolyseprodukt enthält, in die 2. Absorptionsapparatur eingespeist wird, in die der Ozon-führende O₂-Strom, mit der gewünschten Ausgangskonzentration an Ozon eingebracht wird, wodurch in dieser Apparatur ein Ozonüberschuss vorliegt,
worauf nach beendeter Umsetzung das Reaktionsgemisch nach Austritt aus der 2. Absorptionskolonne wiederum in eine Gasphase und eine flüssige Phase getrennt wird, worauf die flüssige Phase, die jetzt nur mehr das entsprechende Ozonolyseprodukt in dem eingesetzten Lösungsmittel enthält, sodann der Aufarbeitungsstufe b) zugeführt wird und der in der Gasphase enthaltene geringe Prozentsatz an Ozon gegebenenfalls in die 1. Absorptionsapparatur, zur weiteren Umsetzung neu eingespeisten Edukts, eingebracht wird.

Durch das erfindungsgemäße Verfahren kann eine Vielzahl von unterschiedlichsten Mono- oder Biscarbonyl- oder Hydroxylverbindungen hergestellt werden.

Beispiele dafür sind Mono- oder Biscarbonyl- oder Hydroxylverbindungen der allgemeinen Formel I 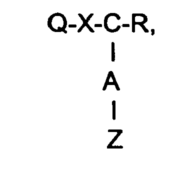 worin
Z entweder OH oder O bedeutet und A für Z gleich OH eine Einfachbindung und für Z gleich O eine Doppelbindung darstellt
Q Wasserstoff oder die Reste, 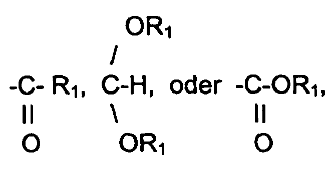 oder -OR₁
bezeichnet, wobei R₁ H bedeutet oder für einen Esterteil steht, der sich von chiralen oder nicht chiralen, primären, sekundären oder tertiären Alkoholen ableitet,
X einen geradkettigen oder verzweigten, ein- oder zweiwertigen, aliphatischen Alkyl- oder Alkylenrest mit 1 bis 50 C-Atomen, wobei dieser Alkyl- oder Alkylenrest durch eine oder mehrere Gruppen, die unter den Reaktionsbedingungen inert sind, substituiert sein kann; einen gegebenenfalls substituierten, geradkettigen oder verzweigten aliphatischen Alkyl- oder Alkylenrest mit 2 bis 50 C-Atomen, wobei eine oder mehrere der -CH₂-Gruppen der Alkyl- bzw. Alkylenkette durch ein Sauerstoffatom, ein Stickstoffatom, ein Schwefelatom oder eine -SO₂-Gruppe ersetzt ist; einen Rest der Formel -(CH₂)ₘ-O-CO-(CH₂)ₚ, wobei m eine ganze Zahl von 1 bis 4 und p eine ganze Zahl von 1 bis 6 sein kann; einen Phenyl- oder Phenylenrest, wobei dieser Phenyl- oder Phenylenrest durch eine oder mehrere Gruppen, die unter den Reaktionsbedingungen inert sind, substituiert sein kann; einen ein- oder zweiwertigen Alkylarylen- oder Alkylenarylenrest mit 7 bis 50 C-Atomen, wobei diese Reste durch eine oder mehrere Gruppen, die unter den Reaktionsbedingungen inert sind, substituiert sein können; einen gegebenenfalls substituierten Heterocyclus mit einem oder zwei Heteroatomen im Ring oder eine Einfachbindung zwischen zwei benachbarten C-Atomen bedeutet, und
R Wasserstoff, einen C₁ bis C₂₀-Alkylrest, -OR₁ oder den Rest 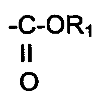 bezeichnet,
oder X und R gemeinsam einen mono- oder bicyclischen Rest mit 4 bis 20 C-Atomen bilden, der ein- oder mehrfach durch unter den Reaktionsbedingungen inerte Gruppen substituiert sein kann.

Unter Esterteil der sich von chiralen oder nicht chiralen Alkoholen ableitet , sind Ester von primären, sekundären oder tertiären Alkoholen zu verstehen. Ester von primären Alkoholen leiten sich bevorzugt von Methanol, Ethanol, Butanol, Propanol oder Hexanol ab. Ester von sekundären oder tertiären Alkoholen leiten sich bevorzugt von acyclischen, monocyclischen, bicyclischen Terpenalkoholen, von acyclischen, monocyclischen, tricyclischen Sesquiterpenalkoholen, Di- oder Triterpenalkoholen, die gegebenenfalls substituiert sein können, ab.

Als Substituenten, die unter den Reaktionsbedingungen inert sind eignen sich beispielsweise folgende Gruppen :
C₁-C₂₀- Alkyl- oder Alkoxy- oder Alkylalkoxygruppen, wie etwa Methyl-, Ethyl-, i-Propyl-, Butyl-, Hexyl-, Octyl- Decyl-, Dodecyl-, Methoxy-, Ethoxy-,Butoxy-, Hexoxy- Methoxymethyl-, Methoxyethyl-, Ethoxymetyl-, Ethoxyethyl-, u.s.w.;
Nitro-, Halogen-, Hydroxyl-, CN-, CONH₂-, Carboxyl-, Carbonsäureester-, Amino-, SO₃H-Gruppen, u.s.w..
Herstellbare Verbindungen sind beispielsweise Benzaldehyd, 4-Methylbenzaldehyd, 3,4-Methylendioxybenzaldehyd, p-Nitrobenzaldehyd, p-Tolualdehyd, Pyridin-4-aldehyd, Pyridin-2-aldehyd, Nonanal, Acetoxyacetaldehyd, Brenztraubensäuremethyl- oder ethylester, α-Ketobuttersäureethylester, Mesoxalsäurediethylester, 3,3-Dimethoxypropanal, 3,3-Di-n-butoxypropanal, Succindialdehyd, Adipinaldehyd, 1,8-Octandial, 3-Thiaglutaraldehyd-3,3-dioxid, Homophtalaldehyd, 1,6-Hexandial-3,4-dicarbonsäuredimethylester, o-Phthalaldehyd, 3-Oxaglutaraldehyd, Glyoxylsäuremethylester-Methanolhalbacetal, Glyoxylsäure-n-butylester-Methanolhalbacetal, Glyoxylsäure-n-octylester-Methanolhalbacetal, Glyoxylsäurementhylester, Glyoxylsäureborneylester, Glyoxylsäurefenchylester, Glyoxylsäure-8-phenylmenthylester, 2-Sulfobenzoesäure, 4-Nitro-2-Sulfobenzoesäure, 4-Nitro-2-sulfobenzaldehyd, 4-Aminobenzoesäure, Therephtalsäure, unsubstituierte oder in Position 4 und/oder 5 und/oder 6 durch C₁-C₄-Alkyl- oder Alkoxy, C₁-C₄Alkyl-C₁-C₄Alkoxy, Halogen Hydroxyl oder Nitro substituierte 2,3-Pyridindicarbonsäuren, 2-Acetylnicotinsäure, Nopinon, Hydroxymethylpyridine, Methyllactat, Butyroxyacetaldehyd u.s.w.

Als Ausgangsverbindungen für die Ozonisierung kommen ungesättigte, organische Kohlenstoffverbindungen mit einer oder mehreren durch Ozon spaltbare olefinische oder aromatische Doppelbindungen im Molekül in Frage.

Dies sind beispielsweise ungesättigte Verbindung der allgemeinen Formel II 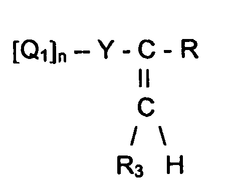 worin n 0 oder 1 ist, Q₁ Wasserstoff oder die Reste 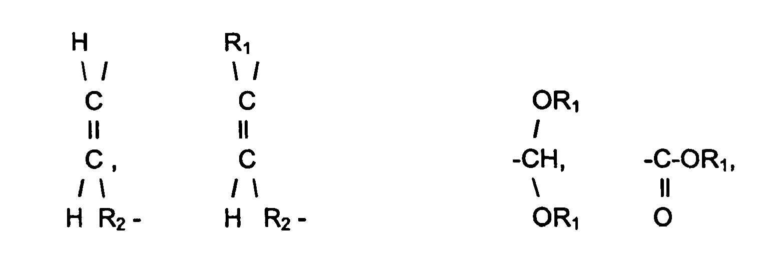 bezeichnet, wobei R₁ wie oben definiert ist,
R₂ und R₃ unabhängig voneinander für Wasserstoff , einen C₁ bis C₄-Alkylrest, einen unsubstituierten oder einen durch unter den Reaktionsbedingungen inerte Gruppen substituierten Phenyl- oder Pyridylrest, oder für einen -COOR₁-Rest stehen, oder einen Rest der Formel (CH₂)ₘ-O-CO-(CH₂)ₚ bedeuten, wobei m eine ganze Zahl von 1 bis 4 und p eine Ganze Zahl von 1 bis 6 sein kann,
oder, falls n 1 ist und Q₁ den Rest 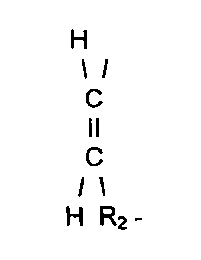 darstellt, stehen R₂ und R₃ zusammen für eine Einfachbindung zwischen zwei benachbarten C-Atomen, oder für einen Alkylen rest mit 2 bis 4 C-Atomen falls Y einen o-Phenylenrest oder einen Alkylenrest mit 2 bis 4 C-Atomen und R ein Wasserstoffatom bedeutet,
ansonsten Y dieselbe Bedeutung wie X in Formel I hat, falls n 1 bedeutet, oder falls n für 0 steht, entweder Wasserstoff bedeutet oder zusammen mit R₃ bzw. mit R₃ und der C=C-Doppelbindung, einen gegebenenfalls substituierten, aliphatischen, araliphatischen, aromatischen oder heteroaromatischen Rest mit 1 bis 50 C-Atomen, der durch Sauerstoff, Stickstoff oder Schwefel durchbrochen sein kann, oder Y mit R₃ und der C=C-Doppelbindung einen gegebenenfalls substituierten mono- oder bicyclischen Rest mit 4 bis 20 C-Atomen, der ein oder 2 Heteroatome aus der Gruppe S, N oder O enthalten kann, bedeutet, oder Y und R gemeinsam einen mono- oder bicyclischen Rest mit 4 bis 20 C-Atomen bilden, der ein- oder mehrfach durch unter den Reaktionsbedingungen inerte Gruppen substituiert sein kann und R wie in Formel I definiert ist.

Geeignete Substituenten sind wiederum C₁-C₂₀- Alkyl- oder Alkoxy- oder Alkylalkoxygruppen, wie etwa Methyl-, Ethyl-, i-Propyl-, Butyl-, Hexyl-, Octyl- Decyl-, Dodecyl-, Methoxy-, Ethoxy-,Butoxy-, Hexoxy- Methoxymethyl-, Methoxyethyl-, Ethoxymetyl-, Ethoxyethyl-, u.s.w.;
Nitro-, Halogen-, Hydroxyl-, CN-, CONH₂-, Carboxyl-, Carbonsäureester-, Amino-, SO₃H-Gruppen, u.s.w..

Als Ausgangsprodukte können demnach solche Verbindungen der Formel II zu den dementsprechenden Mono- oder Biscarbonyl- oder Hydroxylverbindungen der Formel I umgesetzt werden, bei denen beispielsweise für Y unter einem aliphatischen Rest, beispielsweise ein zweiwertiger, geradkettiger oder verzweigter Alkylenrest mit 1 bis 50, bevorzugt 1 bis 20 Kohlenstoffatomen, wobei ein -CH₂-Rest in der aliphatischen Kette durch Sauerstoff, Stickstoff, Schwefel, oder durch den -SO₂-Rest ersetzt sein kann, zu verstehen ist. Als Beispiel für einen araliphatischen Rest sind Aralkylen-, Alkylarylen- oder Alkylenarylenreste mit beispielsweise 7 - 50, bevorzugt 7 - 20 Kohlenstoffatomen, zu verstehen. Beispiel für einen aromatischen Rest ist beispielsweise ein Phenylenrest und für einen heteroaromatischen Rest ein zweiwertiger Rest eines beispielsweise mono- oder bicyclischen Heterocyclus mit einem oder zwei Heteroatomen im Ring, wobei die Ringe bevorzugt fünf- oder sechsgliedrigen sind. Die vorgenannten Reste können noch durch eine oder mehrere unter den Reaktionsbedingungen inerte Gruppen, beispielsweise durch Alkyl-, Alkoxy- oder Alkoxycarbonylgruppen mit jeweils 1 bis 10 Kohlenstoffatomen, bevorzugt mit 1 bis 4 Kohlenstoffatomen, oder durch Nitrogruppen substituiert sein.

In bevorzugter Weise werden ungesättigte Verbindungen der Formel IIa 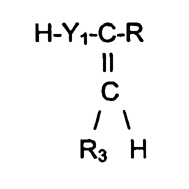 in der
R wie in Formel I und R₃ wie in Formel II definiert ist und
Y₁ und R₃ gleich sind und beide den Rest -(CH₂)ₘ-O-CO-(CH₂)ₚ mit m gleich 1 oder 2 und
p gleich 1, 2 oder 3 bedeuten, oder
Y₁ zusammen mit Wasserstoff einen gegebenenfalls in ortho- und/oder meta- und/oder para-Stellung substituierten Phenylrest oder einen gegebenenfalls substituierten fünf- oder sechsgliedrigen Heteroarylrest mit einem Heteroatom im Ring, besonders bevorzugt aber den para-Nitrophenyl-, p-Tolyl, 2- oder 4-Pyridinylrest oder gemeinsam mit der C=C-Doppelbindung einen gegebenenfalls substituierten mono- oder bicyclischen Heterocyclus, wie beispielsweise unsubstituiertes oder substituiertes Chinolin oder Indol, darstellt, oder in der Y₁ und R gemeinsam einen bicyclischen Rest mit 4 bis 10 C-Atomen bilden, der ein- oder mehrfach durch unter den Reaktionsbedingungen inerte Gruppen substituiert sein kann zu den dementsprechend bevorzugten Carbonyl- bzw. Hydroxylverbindungen umgesetzt.

Beispiele für ungesättigte Verbindungen der Formel IIa sind Butendiol(1,4)dibutyrat, para-Nitro- oder para-Methylstyrol, 2- oder 4-Vinylpyridin, Chinolin, 8-Methylchinolin, 3-Ethyl-8-methylchinolin, Indol, Thiophendioxid, Stilben-2,2'-disulfonsäure, 4,4'-Dinitrostilben-2,2'disulfonsäure, 4,4'-Vinylendianilin, 4,4'-Vinylendipyridin, 4,4'-Stilbendicarbonsäure, β-Pinen.

Bevorzugt werden auch ungesättigte Verbindungen der Formel IIb 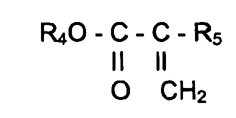 in der
R₄ Methyl oder Ethyl und R₅ Methyl, Ethyl oder den Ethoxycarbonylrest bezeichnen, zu den dementsprechend bevorzugten Carbonylverbindungen umgesetzt. Ganz besonders bevorzugt umgesetzt werden Verbindungen in denen R₄ und R₅ Methyl bedeutet. Beispiele für Ausgangsverbindungen der Formel IIb sind Methylmethacrylat, Alkylacrylsäureethylester oder Diethylmethylenmalonat.

Eine weitere bevorzugte Gruppe an Ausgangsprodukten zur Herstellung der dementsprechend bevorzugten Carbonylverbindungen der Formel I sind Verbindungen der Formel IIc 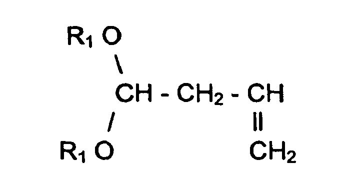 in der R₁, wie in Formel I definiert ist. Beispiele für Verbindungen der Formel IIc sind 4,4-Dimethoxybuten oder 4,4-Di-n- butoxy-buten.

Weiters werden in bevorzugter Weise Verbindungen der Formel IId

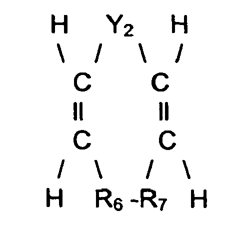

in der Y₂ einen o-Phenylenrest oder einen Alkylenrest mit 2 bis 4 C-Atomen und R₆ und R₇ gemeinsam eine Einfachbindung zwischen den benachbarten C-Atomen oder einen Alkylenrest mit 2 bis 4 C-Atomen bezeichnen, zu den dementsprechend bevorzugten Dialdehyden der Formel I umgesetzt. Beispiele für Verbindungen der Formel IId sind Naphthalin oder Cyctooctadien ( 1.5).
Schließlich wird in bevorzugter Weise eine weitere Gruppe von ungesättigten Verbindungen der Formel IIe 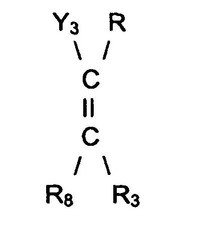 in der, wenn R und R₃ jeweils H bedeuten, Y₃ und R₈ zusammen einen Alkylenrest mit 2 bis 6 C-Atomen oder die Reste -CH₂-SO₂-CH₂-, -CH₂-O-CH₂, 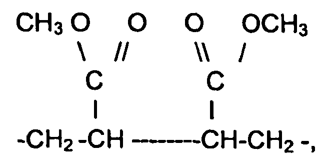 oder 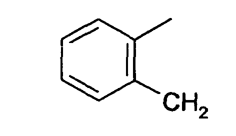 bedeuten, zu den dementsprechend bevorzugten
Dialdehyden der Formel I umgesetzt, beziehungsweise wenn R und R₃ jeweils COOR₁ und Y₃ und R₈ H bedeuten zu den dementsprechend bevorzugten Glyoxylsäureestern, deren Halbacetale oder Monohydrate der Formel I.

Beispiele für Verbindungen der Formel IIe sind Cyclohexen, Cycloocten, Cyclododecen, Sulfolen, Inden, Tetrahydrophthalsäuredimethylester oder 2,5-Dihydrofuran, sowie Maleinsäuredimethyl- oder -diethylester, Maleinsäuremonophenylmenthylester, Maleinsäuremonomenthyl-, fenchyl- oder boneylester, sowie die analogen Fumarsäureester.

Für das erfindungsgemäße Verfahren eignen sich somit die unterschiedlichsten Verbindungen, die auch komplexe Strukturen mit den unterschiedlichsten Funktionalitäten enthalten können. Neben den bereits erwähnten bevorzugten Ausgangsverbindungen eignen sich somit auch Verbindungen mit komplexen Strukturen, wie etwa Cephalosporine u.s.w. als Edukt. Einzige Voraussetzung bzw. Einschränkung bei der Auswahl der Edukte ist das Vorhandensein mindestens einer durch Ozon spaltbaren Doppelbindung.

Die erfindungsgemäße Ozonisierung wird bei Temperaturen von -80°C bis knapp unterhalb der Explosionsgrenze des verwendeten Lösungsmittels, d.h. in Abhängigkeit vom verwendeten Lösungsmittel bis 100°C durchgeführt. Vorzugsweise beträgt die Temperatur, wiederum in Abhängigkeit vom eingesetzten Lösungsmittel -30 bis +80°C, wobei die Einhaltung einer Temperatur von -20 bis +50°C wiederum besonders bevorzugt ist. Die Ozonolyse kann dabei bei Normaldruck oder unter Druck erfolgen.

Die Umsetzung der ungesättigten Verbindungen mit Ozon in Stufe a) erfolgt in einem organischen Lösungsmittel, in dem die Ausgangsverbindungen gut löslich sind oder in einer wässrigen Lösung.

Als organische Lösungsmittel kommen demnach Alkohole, Carbonsäuren, Kohlenwasserstoffe u.s.w. in Frage.

Bevorzugte Lösungsmittel sind niedere aliphatische Alkohole mit 1 bis 6 C-Atomen, wie Methanol, Ethanol, i-Propanol, u.s.w., wobei die Verwendung von Methanol und Ethanol besonders bevorzugt ist, oder Gemische mit nicht halogenierten Kohlenwasserstoffen.

Bei der Herstellung von beispielsweise Glyoxylsäureesterhalbacetalen der Formel I, kommt dem als Lösungsmittel verwendeten Alkohol insofern Bedeutung zu, als dieser Alkohol an der Acetalbildung teilnimmt.

Der Ozonisierungsschritt kann jedoch auch in Abhängigkeit vom eingesetzten Edukt in wässriger Lösung durchgeführt werden. Ist die Ausgangsverbindung selbst nicht wasserlöslich, werden deren Salze eingesetzt. Dabei eignen sich alle Salze, die zu wasserlöslichen Verbindungen führen. Beispiele dafür sind Alkali- oder Erdalkalisalze, wie etwa Natrium-, Kalium-, Kalzium- oder Magnesiumsalze. Es kann jedoch auch durch Zusatz einer geeigneten Säure oder Base, die wässrige Lösung des entsprechenden Salzes der gewählten Ausgangsverbindung hergestellt werden. Als Säuren werden bevorzugt Mineralsäuren, wie Schwefelsäure, Salpeter- oder Phosphorsäure verwendet.

Die Umsetzung mit Ozon erfolgt erfindungsgemäß kontinuierlich, wobei Ozon je nach Reaktivität der Edukte bzw. Substrate im Verhältnis zum eingesetzten Lösungsmittel, in stöchiometrischen Mengen bis zu einem 40%igen Überschuss verwendet wird. Bevorzugt wird mit stöchiometrischen Mengen bis zu einem bis zu 20%igen Überschuss an Ozon gearbeitet.

In einer ersten Variante wird eine Vorrichtung eingesetzt, die aus zwei Absorptionsapparaturen, Vorrichtungen zur Abfuhr der Reaktionswärme, wie etwa externen oder internen Wärmetauschern, und Vorrichtungen zum Trennen von Gas- und Phlüssigphase, besteht.

Die Eduktströme sind dabei gegenläufig. Das Ausgangsprodukt wird in die erste Absorptionsapparatur eingespeist, wobei die Ausgangskonzentration vom eingesetzten Edukt und den Reaktionsbedingungen abhängt und bevorzugt zwischen 1 und 3 mol/l, bezogen auf die Doppelbindungen, besonders bevorzugt zwischen 1,2 und 2 mol/l, bezogen auf die Doppelbindungen, liegt; der Ozon-führende O₂-Strom wird hingegen in die zweite Absorptionsapparatur eingebracht. Die Ozonmenge wird dabei in Abhängigkeit von der Reaktivität des Eduktes so gewählt, dass sie für reaktive Substanzen bevorzugt einem beinahe stöchiometrischem Ozonverbrauch bis zu einem etwa 107% der stöchiometrischen Menge und für weniger reaktive Substanzen einem Ozonverbrauch von etwa 107 bis 140%, bevorzugt bis 120%, der stöchiometrischen Menge bezogen auf die Ausgangsverbindung entspricht.

In der 1. Absorptionsapparatur wird das eingesetzte Edukt mit dem Ozonstrom in Kontakt gebracht, der nach Durchlauf der 2. Absorptionsapparatur, in die 1. Absorptionsapparatur eingespeist wird. In dieser Apparatur liegt ein Ozonunterschuss vor, da hier große Mengen an Edukt vorliegen, während der Ozongehalt des eingespeisten Stromes durch Abreagieren in der 2. Absorptionsapparatur je nach Substrat und Kolonnenbeschaffenheit um bis zu 95% reduziert wird. Nach der Reaktion des in die 1. Absorptionsapparatur eingespeisten Ozons mit dem entsprechend eingebrachten Edukt, tritt das Reaktionsgemisch aus der 1. Apparatur aus und wird in eine Gasphase und eine flüssige Phase getrennt. In der Gasphase ist praktisch kein Ozon mehr enthalten. Die flüssige Phase, die jetzt noch nicht umgesetztes Edukt, Lösungsmittel und das entsprechende Ozonolyseprodukt enthält, wird sodann in die 2. Absorptionsapparatur eingespeist, in die, wie bereits oben beschrieben, der Ozon-führende O₂-Strom, mit der angegebenen Ausgangskonzentration an Ozon eingebracht wird. Somit liegt in dieser Apparatur ein Ozonüberschuss, bezogen auf das Edukt, vor, da nur noch ein geringer Prozentsatz an Eduktmenge, bezogen auf die ursprünglich eingesetzte Menge vorliegt. Die Eduktmenge beträgt demnach lediglich nur mehr 1 bis 10%, bevorzugt 1 bis 5% und besonders bevorzugt 1 bis 3 % der Ausgangskonzentration. Im Gegensatz dazu liegen in der 2. Absorptionsapparatur bereits große Mengen an Ozonolyseprodukt, erhalten durch die Reaktion in der 1. Absorptionsapparatur, vor.

Unerwarteterweise reagiert das zugeführte Ozon trotz der geringen Konzentration an Edukt und der hohen Konzentration an Ozonolyseprodukt schneller mit den Restmengen an Edukt als mit dem Ozonolyseprodukt oder dem Lösungsmittel, sodass es auch bei diesen Konzentrationsverhältnissen nicht zu den eigentlich vom Fachmann erwarteten hohen Ausbeuteverlusten kommt.

Nach beendeter Umsetzung tritt das Reaktionsgemisch aus der 2. Absorptionsapparatur aus und wird wiederum in eine Gasphase und eine flüssige Phase getrennt. In der Gasphase ist, wie oben beschrieben, nur noch ein geringer Prozentsatz an Ozon enthalten, das in die 1. Apparatur, zur weiteren Umsetzung neu eingespeisten Edukts, eingebracht wird. Die flüssige Phase, die jetzt nur mehr das entsprechende Ozonolyseprodukt in dem eingesetzten Lösungsmittel enthält, wird sodann der Aufarbeitungsstufe (Hydrierung, Oxidation oder Erwärmen) zugeführt.

Diese Variante wird bevorzugt für im Vergleich zum Lösungsmittel oder zum gebildeten Ozonolyseprodukt schnell reagierende Edukte angewandt.

In einer weiteren Variante wird wiederum der Eduktstrom in die 1. Absorptionsapparatur eingeleitet. Der Ozon-führende O₂-Strom wird bei dieser Verfahrensweise gleich in die 1. Apparatur eingebracht, wobei aber ein Teil des Stromes gleich für die 2. Apparatur abgezweigt und in diese eingespeist wird (Ozon-split). Die Aufteilung des Ozon-führende O₂₋Stromes erfolgt dabei in einem Verhältnis von 1. Apparatur zu 2. Apparatur von 50 : 50 bis 90 : 10, bevorzugt von 70 : 30 bis 85 : 15 .

Der in Apparatur 1 und 2 eingespeiste O₂-Strom enthält dabei etwa 4-10%, bevorzugt 5-8% Ozon. In der 1. Absorptionsapparatur liegt demnach wieder ein Ozonunterschuss vor, da in dieser Apparatur große Mengen an Edukt vorhanden sind. Die Eduktkonzentration, bezogen auf die Doppelbindungen, nach Durchlauf der 1.Absorptionsapparat hängt von dem Aufteilungsverhältnis des Ozonstromes ab und beträgt bei einer Aufteilung von 50:50 bevorzugt 0,9 bis 2 mol/l und besonders bevorzugt 1 bis 1,5 mol/l und bei einer Aufteilung von 90:10 bevorzugt 0,1 mol/l bis 0,5 mol/l, besonders bevorzugt 0,1 bis 0,3 mol/l.

Nach beendeter Umsetzung tritt das Reaktionsgemisch aus der 1.Absorptionapparatur aus und wird in eine Gasphase und eine flüssige Phase getrennt, wobei in der Gasphase nur noch ein geringer Prozentsatz an Ozon enthalten ist. Die flüssige Phase, die hauptsächlich das entsprechende Ozonolyseprodukt in dem eingesetzten Lösungsmittel und restliches, noch nicht umgesetztes Edukt in einer Konzentration von nur mehr 5 bis 50%, bevorzugt 10 bis 50% der Ausgangskonzentration enthält, wird sodann in die 2. Absorptionsapparatur eingebracht, wo sie mit dem, wie oben beschrieben abgezweigten Ozonstrom in Kontakt gebracht wird. In der 2. Absorptionsapparatur liegt ein Ozonüberschuss vor, da wie beschrieben nur noch geringe Mengen an Edukt in die 2. Apparatur gelangen. Trotzdem Ozonolyseprodukt und Lösungsmittel, die eigentlich eine hohe Neigung zur Reaktion mit Ozon haben, in wesentlich größeren Mengen verglichen mit dem Edukt vorliegen, reagiert dennoch das noch nicht umgesetzte Edukt mit dem eingespeisten Ozon.

Nach beendeter Umsetzung tritt das Reaktionsgemisch aus der 2.Absorptionsapparatur aus und wird wiederum in eine Gasphase und eine flüssige Phase getrennt. In der Gasphase sind keine oder nur mehr vernachlässigbar kleine Mengen an Ozon enthalten. Die flüssige Phase, die jetzt nur mehr das entsprechende Ozonolyseprodukt in dem eingesetzten Lösungsmittel enthält, wird sodann der Aufarbeitungsphase (Schritt b) zugeführt.

Diese Variante wird bevorzugt für langsam reagierende Substrate angewandt.

In einer Abwandlung dieser Variante kann das Abgas aus der 1. Absorptionsapparatur dem abgezweigten Teilstrom zugemischt werden, wodurch dieser verdünnt wird. In diesem Fall wird eine Sauerstoffeinsparung beim Ozon-führenden O₂-Strom erzielt. Außerdem wird der Gesamtozonverbrauch etwas gesenkt.

Es ist weiters möglich, nur den Abgasstrom aus der 1. Absorptionsapparatur in die 2. Apparatur einzubringen und auf die Teilung des ursprünglichen Ozon-führenden O₂-Stromes zu verzichten, sodass der gesamte Ozon-führende O₂-Strom in die 1. Apparatur eingeleitet wird. Dadurch wird sowohl der Ozonunterschuss in der 1. Absorptionsapparatur, als auch der Ozonüberschuss in der 2. Apparatur verringert.

Bei allen Ozonisierungsvarianten ist es auch möglich, Edukt bei ununterbrochener Ozonisierung nachzudosieren, sobald der Gehalt an Edukt auf einen vorher festgelegten Wert gesunken ist, sodass der Gehalt auf diesem Wert während der Ozonisierung konstant gehalten wird.

Unter Absorptionsapparaturen sind bei der erfindungsgemäßen Ozonisierung übliche Apparaturen, die einen Gas-Flüssigaustausch bewerkstelligen, wie etwa Absorptionskolonnen, Blasensäulen, gerührte Reaktoren, Rührkessel, Mischer, Schleifenreaktoren u.s.w. zu verstehen.

In einer weiteren bevorzugten Ausführungsform wird die kontinuierliche Ozonolyse in zwei Blasensäulen als Absorptionsapparaturen durchgeführt. Der Ozonstrom kann dabei wieder gegenläufig, wie in der Variante für schnellreagierende Substrate, geführt werden, bevorzugt wird aber der Ozon-split angewandt.

Die Kombination von Blasensäulen als Absorptionsapparaturen und Ozon-split eignet sich besonders für langsamreagierende Substrate und für Reaktionen im wässrigen System, beispielsweise für die Ozonolyse von Chinolin im wässrigen System.

Die erfindungsgemäße kontinuierliche Ozonolyse zeichnet sich durch ihre einfache Prozessführung aus. Von Vorteil ist insbesondere, dass es zu keinen Ozonunterbrechungen bei Chargenwechseln kommt und die Ozonmenge bzw. -konzentration beim Durchbruch, d.h. beim Reaktionsende leichter kontrollierbar ist. Durch diese kontinuierliche Fahrweise werden außerdem die Peroxidmengen in der Reaktionslösung im Vergleich zum Stand der Technik kleiner gehalten. Das die Umlaufapparatur für die Ozonolyse verlassende Reaktionsgemisch weist einen Peroxidgehalt von 1 bis 2, bevorzugt von 1 bis 1,5 mol/l auf.

Im Anschluss an die kontinuierliche Ozonolyse erfolgt erfindungsgemäß die Aufarbeitung der Peroxidlösung, die von den gewählten Reaktionsbedingungen während der Ozonolyse abhängt. Wird die Ozonolyse in wässriger oder mineralsaurer, wässriger Lösung durchgeführt, so können die durch die Ozonolyse erhaltenen Peroxide beispielsweise durch einfaches Erwärmen in die entsprechenden Endprodukte überführt werden. Dies ist insbesondere der Fall, wenn substituierte Chinoline in die entsprechenden substituierten Pyridincarbonsäuren, wie etwa in 2-Acetylnicotinsäure, überführt werden. Bevorzugt wird dabei gleichzeitig Sauerstoff, in Form von reinem Sauerstoff oder in Form von Luft, eingeblasen, sodass die Bildung von Nebenprodukten verhindert wird.

In anderen Fällen ist im Anschluss an die Ozonolyse ein Oxidationsschritt notwendig, um zu den gewünschten Endprodukten zu gelangen. Dazu wird die Peroxidlösung mit einem geeigneten Oxidationsmittel, beispielsweise mit Wasserstoffperoxid, Hypochlorid, Persäuren, Peroxodisulfat, u.s.w., versetzt.

Werden Stilbenverbindungen als Ausgangsprodukte eingesetzt, so liegt nach der Ozonolyse ein Gemisch aus entsprechendem Aldehyd und Hydroperoxid vor. Das Peroxid kann sowohl sauer als auch alkalisch zersetzt werden. Ist der Aldehyd das gewünschte Endprodukt, so wird dieser aus dem Gemisch isoliert. Ist die korrespondierende Säure das gewünschte Produkt, erfolgt noch ein Oxidationsschritt.

Erfolgt die Ozonolyse nicht in wässriger Lösung, sondern in einem organischen Lösungsmittel, so wird im Anschluss an die Ozonolyse eine kontinuierliche Hydrierung durchgeführt. Dabei ist es lediglich maßgeblich, dass die peroxidischen Ozonolyseprodukte in einem unter den Reaktionsbedingungen der Hydrierung inerten, organischen Verdünnungsmittel zumindest teilweise gelöst vorliegen. Unter organischen Verdünnungsmitteln sind dabei neben den in der nicht-wässrigen Ozonolyse verwendeten Lösungsmittel, übliche, bei der Hydrierung verwendete Verdünnungsmittel zu verstehen, wie beispielsweise aliphatische oder aromatische, gegebenenfalls chlorierte Kohlenwasserstoffe, wie Pentan, Hexan, Cyclohexan, Toluol, Xylole, Methylenchlorid, Dichlorethan, Chlorbenzole, Carbonsäureester wie Essigsäuremethyl-, -ethyl- oder - butylester, Ether und Ketone, sofern sie nicht zur Bildung sicherheitstechnisch bedenklicher Peroxide fähig sind, sowie Alkohole wie Methanol, Ethanol, iso-Propanol. Bei der Verwendung von Alkoholen als Verdünnungsmittel können als Produkte nicht nur die, den eingesetzten Olefinen entsprechenden, Aldehyde oder Ketone, sondern auch deren Halbacetale, Acetale oder deren Ketale entstehen, wobei die Acetalisierung oder Ketalisierung im wesentlichen von den pH-Wert Bedingungen abhängig ist.

Bevorzugt werden im erfindungsgemäßen Verfahren peroxidische Ozonolyselösungen in einem niederen, aliphatischen Alkohol mit 1 bis 6 C-Atomen, besonders bevorzugt in Methanol oder Ethanol, eingesetzt. Die Konzentration der Peroxide in der Lösung ist für das erfindungsgemäße Verfahren aber überraschenderweise nicht von Bedeutung. Im allgemeinen weisen die Lösungen der peroxidischen Ozonolyseprodukte, die durch die oben beschriebene, kontinuierliche Ozonolyse erhalten werden, eine Peroxidkonzentration von unter 2, bevorzugt von unter 1,5 Mol/l auf. Da Peroxide in höheren Konzentrationen zur explosionsartigen Zersetzung neigen, wird deshalb bevorzugt darauf geachtet, dass die eingesetzten Lösungen eine Peroxidkonzentration unter 2, besonders bevorzugt unter 1,5 Mol/l aufweisen.

Die an die Ozonisierung anschließende katalytische Hydrierung der Ozonolyseprodukte wird beim erfindungsgemäßen Verfahren in verdünnter Lösung durchgeführt , wobei gegebenenfalls durch geeignete Maßnahmen und Vorrichtungen dafür Sorge getragen wird, dass während der gesamten Hydrierung in der Hydrierlösung ein Peroxidgehalt von unter 1,5 Mol/l, bevorzugt von unter 1 Mol/l, besonders bevorzugt von unter 0.1 Mol/l, ganz besonders bevorzugt von höchstens 0.05 Mol/l und insbesondere von höchstens 0.02 Mol/l eingestellt und aufrecht erhalten wird.

Zur praktischen Durchführung wird beispielsweise in einem Hydrierreaktor eine Suspension des Katalysators in dem in Stufe a) bei der Ozonisierung verwendeten Alkohol, bevorzugt in Methanol oder Ethanol, ganz bevorzugt in Methanol vorgelegt und die bei der Ozonisierung erhaltene Lösung mittels einer regelbaren Dosiervorrichtung kontinuierlich eingespeist. Bei der Zugabe der Ozonolyselösung zu Beginn und im Verlaufe der Hydrierung ist selbstverständlich darauf zu achten, dass durch die zugeführte Menge der peroxidhältigen Ozonisierungsprodukte der oben angegebene Peroxidgehalt in der Hydrierlösung nicht überschritten wird.

Durch die geringe Konzentration an peroxidhältigen Ozonisierungsprodukten während des eigentlichen Hydriervorganges ist das Mengenverhältnis von Katalysator zum zu reduzierendem Substrat über die gesamte Dauer der Hydrierung hinweg gleichmäßig günstig, sodass auch bei sparsamen Einsatz des Katalysators eine rasche Reduktion gewährleistet ist. Auf diese Weise wird auch die bei hohen Peroxidkonzentrationen sonst zu beobachtende Vergiftung und der damit verbundene Aktivitätsverlust des Katalysators verhindert.

Im Gesamten gesehen kann aber durch die kontinuierliche Einspeisung eine große Menge an Ozonisierungsprodukten in einem verhältnismäßig kleinen Volumen reduktiv aufgespalten werden, wodurch in der Endstufe des Verfahrens konzentrierte Lösungen anfallen und neben Lösungsmittel selbst auch Zeit und Kosten bei der destillativen Entfernung der Lösungsmittel während der Aufarbeitung gespart werden.

Als Katalysatoren eignen sich die für Hydrierungen üblicherweise verwendeten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet, insbesondere Platinkatalysatoren ohne Trägermaterial. Bei Pulverkontakten eignen sich als Trägermaterial beispielsweise Kohle, Aluminium, Silikagel oder Kieselgur. Es können auch Monolithkatalysatoren verwendet werden. Unter einem Monolithkatalysator ist ein Katalysator zu verstehen, die aus einem Träger, der mit einem Katalysatorgrundstoff beschichtet ist, bestehen. Der Träger besitzt bevorzugt eine möglichst große Oberfläche, die beispielsweise durch waben-oder lamellenförmige Strukturierung erreicht werden kann. Der Träger liegt in einem Stück vor und kann aus dazu geeigneten Materialien, beispielsweise aus Metall, Glas, Keramik, Kunststoff bestehen. Bevorzugt ist ein Metallträger, beispielsweise aus Stahl, Aluminium, da sich gezeigt hat, dass dieser die Reaktionswärme gleichmäßig aufnehmen und wieder in das umgehende Reaktionsmedium abgeben kann. Es hat sich nämlich herausgestellt, dass es bei Verwendung nichtleitender Materialien als Träger zu lokalen Überhitzungen im Reaktionsmedium kommen kann, sodass Ausbeuten und Reinheit der Reaktionsprodukte negativ beeinflußt werden können. Als Katalysatorgrundstoff sind bei der Reduktion organischer Peroxidlösungen wiederum übliche Katalysatorgrundstoffe zu verstehen. Übliche Katalysatorgrundstoffe sind beispielsweise Edelmetalle wie Platin, Palladium, Übergangsmetalle, wie Nickel, Kobalt, Rhodium, deren Oxide, oder Mischungen solcher Metalle oder Metalloxide. Dabei können diese Metalle durch Schwermetalle wie Blei, Wismuth partiell vergiftet sein. Bevorzugt werden im erfindungsgemäßen Verfahren Edelmetalle oder Mischungen von Edelmetallen mit Übergangsmetallen als Katalysatorgrundstoff eingesetzt. Die Ausbeuten sind beim erfindungsgemäßen Verfahren an sich unabhängig von der eingesetzten Katalysatormenge, jedoch empfiehlt sich zur Erzielung einer ausreichenden Hydriergeschwindigkeit die genannten Katalysatoren, in Edelmetallmengen von 0.1 bis 5 Gew.-%, vorzugsweise von 0.5 bis 2 Gew.-%, bezogen auf die jeweils pro Stunde eingespeiste Gesamtmenge an Ozonisierungsprodukten, vorzulegen.

Beim erfindungsgemäßen Verfahren werden zur Reduktion der Ozonisierungsprodukte äquivalente Mengen an Wasserstoff verbraucht. Die Menge an Wasserstoff, die bei der Hydrierung verwendet werden kann, reicht von einem Moläquivalent bis zu einem mehrfachen molaren Überschuß. Die Verwendung von überschüssigem Wasserstoff bringt an sich keine Vorteile und ist nur zweckmäßig, um eine ausreichende Versorgung des Hydriergemisches mit Wasserstoff sicherzustellen.

Die Hydrierung kann beim erfindungsgemäßen Verfahren unter praktisch drucklosen Bedingungen erfolgen. Unter praktisch drucklosen Bedingungen sollen hier Drücke von 1 bis etwa 3 bar verstanden werden, wie das in der Technik üblich ist, um das Eindringen von Luft in den Hydrierreaktor zu verhindern. Auf diese Weise ist die Reduktion der Ozonisierungsprodukte in technischer und apparativer Hinsicht sehr einfach durchzuführen. Es ist aber auch möglich, die Hydrierung bei einem Druck bis zu 20 bar durchzuführen und dadurch die Hydrierungsgeschwindigkeit zu steigern.

Die reduktive Spaltung verläuft im allgemeinen exotherm und wird in Abhängigkeit vom Produkt bei Temperaturen von -10 bis +150°C, gemäß einer bevorzugten Ausführungsform der vorliegenden Erfindung bei +15 bis +70°C und besonders bevorzugt bei Temperaturen im Bereich von +20 bis +50°C durchgeführt.

Bevorzugt wird während der Hydrierung ein pH-Wert von 2 bis 5 eingehalten. Da sich im Verlaufe der Hydrierung in geringen Mengen saure Nebenprodukte bilden können, kann gegebenenfalls zur Einhaltung des gewünschten pH-Wertes die dosierte Zugabe einer Base, bevorzugt von verdünnter Natronlauge, erfolgen.

Nach Beendigung der Hydrierung erhält man unter den Bedingungen des erfindungsgemäßen Verfahrens eine bevorzugt alkoholische Lösung der Verfahrensprodukte die gänzlich peroxidfrei ist und in gefahrloser Weise aufgearbeitet werden kann.

Für die erfindungsgemäße kontinuierliche Hydrierung eignen sich alle Hydrierreaktoren, die einen ausreichenden Massetransfer von Wasserstoff in die Flüssigphase gewährleisten.

Dies können beispielsweise Rührreaktoren verschiedenster Bauart, wie beispielsweise Rührkessel, Schleifenreaktor, u.s.w., die geeignete Rührer, wie etwa 3-flügelige Rührer, Injektoren u.s.w. aufweisen. Es können aber auch gerührte oder ungerührte Blasensäulen, Festbettreaktoren u.s.w. eingesetzt werden.

Bei dem erfindungsgemäßen Verfahren wird dabei in einer Variante die aus der Ozonolysestufe erhaltene peroxidhaltige Ozonolyseproduktlösung und der Wasserstoffstrom in die Hydrierapparatur eingeleitet. Die Hydrierapparatur besteht dabei beispielsweise aus einem Rührreaktor, ausgerüstet mit einem 3-flügeligem Rührer, Wasserstoffeinleitung, Wasserstoffmessung, pH-Messung, Temperaturmessung, Kühlung, Filtrationseinrichtung und Dosierpumpen. Das gewünschte Lösungsmittel und der eingesetzte Katalysator, bevorzugt kein Monolythkatalysator, werden vorgelegt. Die Peroxidlösung wird sodann unter Rühren, bevorzugt unter kräftigem Rühren bei kontinuierlicher Wasserstoffgaseinleitung eingespeist. Über die Geschwindigkeit der Dosierung kann der gewünschte Peroxidgehalt geregelt werden. Gegebenenfalls kann gleichzeitig der basische Zusatz zur Regelung des pH-Wertes eingebracht werden. Das Volumen der Reaktorlösung wird durch standgeregelten Austrag über die Filtrationseinheit konstant gehalten, wobei der Peroxidgehalt der ausgetragenen Lösung laufend kontrolliert wird. Der Peroxidgehalt der abgetrennten Lösung liegt dabei unter 0,01 mo/l. Ein Abtrennen vom Katalystor entfällt hier im Gegensatz zum Stand der Technik, da der Katalysator bei geeigneter Wahl der Filtrationseinheit nicht mit der Produktlösung ausgetragen wird, sondern wieder in das Reaktionsgefäß rückgeführt wird. Besonders geeignete Filtrationseinheiten sind demnach Querstromfiltrationsapparaturen, die beispielsweise mit Metallfritten in Form von Sinterrohren ausgestattet sind, oder getauchte Metallfritten.

Durch die erfindungsgemäße Verfahrensführung ist es somit möglich den Katalysator über Jahre hinweg zu gebrauchen, da keine chemische Vergiftung erfolgt. Lediglich mechanische Abnützungen machen sich bei jahrelanger Benutzung bemerkbar. Weiters werden die Peroxide schnell und zuverlässig abreagiert.

Schritt b) kann sowohl kontinuierlich, aber auch diskontinuierlich durchgeführt werden.

### Beispiel 1: (Vergleichsbeispiel A: Batch)

### a) Ozonisierung:

In eine kontinuierliche Umlaufapparatur, bestehend aus einer Absorptionskolonne, Trenngefäß, Umwälzpumpe und externem Wärmetauscher wurden 4 Liter einer methanolischen Lösung von 900 g DMM (Gehalt von 225 g/l, entsprechend 1,56 mol/l) vorgelegt. Die Temperatur wurde durch Kühlung über den externen Wärmetauscher auf -20 °C gekühlt. Die Kreislaufmenge betrug ca. 220 I/h.

Die Lösung wurde in der Absorptionskolonne mit 2500 Nl/h Ozon/Sauerstoffstrom mit einem Ozongehalt von 55 g/Nm3 in Kontakt gebracht und reagierte mit dem enthaltenen Ozon. Die exotherme Reaktion fand praktisch sofort statt, das Ozon wurde quantitativ aufgenommen. Im Trenngefäß am Fuß der Absorptionskolonne trennte sich das Gemisch in eine Flüssigphase und eine Gasphase.

Nach beendeter Ozonolyse betrug der DMM Gehalt ca. 2 g/l entsprechend 1 % der Ausgangsmenge.

Die aufgenommene Ozonmenge wurde bestimmt und betrug insgesamt ca. 305 g entsprechend 102% der Theorie.

### b) Hydrierung:

Die bei der Ozonolyse erhaltene Lösung wurde portioniert und über ein Dosiergefäß in einen Hydrierreaktor, in dem eine Suspension von 1,5 g Pt Adams Katalysator, hergestellt durch Hydrierung von PtO₂, in 0,5 Liter Methanol vorgelegt wurde und der mit Wasserstoff gefüllt wurde, in solchen Dosen eingespeist, dass der Peroxidgehalt im Hydrierreaktor zu Beginn und im Verlaufe der gesamten Hydrierung maximal 0,1 Mol/l betrug. Unter kräftigem Rühren und Wasserstoffzugabe wurde bis zur negativen Peroxidprobe weiterhydriert, wobei über die gesamte Hydrierperiode eine Temperatur von 30°C - 33 °C und durch Zugabe von methanolischer NaOH ein pH- Wert von 2 bis 4 eingehalten wurde.

Anschließend wurde der Inhalt des Hydrierreaktors bis auf einen Rückstand von 0,5 Liter über eine Fritte abgesaugt, von Neuem ozonisierte Lösung über das Dosiergefäß in den Reaktor eingespeist und der Hydriervorgang unter den oben angegebenen Reaktionsbedingungen wiederholt.

Nach Beendigung der Hydrierung wurde ein polarographisch bestimmter Glyoxylsäuremethylester- Methanolhalbacetalgehalt von 12,125 mol (97% der Theorie) festgestellt. Zur Weiterverarbeitung wurde in gebundener Form im Hydrierungsgemisch vorhandene NaOH unter Kühlung vorsichtig mit 98%iger H₂SO₄ als Na₂SO₄ ausgefällt und durch Filtration abgetrennt. Das Methanol wurde dann am Rotavapor entfernt und der Rückstand bei etwa 55°C und 25 Torr destilliert. Die Ausbeute an reinem Glyoxylsäuremethylester - Methanolhalbacetal betrug 1425 g (11,87 Mol), entsprechend 95% der Theorie.

### Beispiel 2:

### a) Ozonisierung

In der Umlaufapparatur aus Beispiel 1 wurden 4 Liter methanolische DMM Lösung wie in Beispiel 1 beschrieben ozonisiert. Sobald der DMM Gehalt auf 2 g/l abgesunken war, wurde bei ununterbrochener O₃ Einleitung weitere DMM Lösung mit derselben Konzentration wie in Beispiel 1 so eingespeist, dass der DMM Gehalt zwischen 2 und 3 g/Liter konstant gehalten wurde. Auf diese Weise wurden insgesamt 16 Liter methanolische Peroxidlösung erhalten, es wurden 3600 g DMM ozonisiert.

Die insgesamt aufgenommene Ozonmenge betrug 1450 g entsprechend 30,25 mol = 121% der Theorie. Der Ozonverbrauch ist gegenüber Beispiel 1 wesentlich erhöht, Ozon wurde in Nebenreaktionen verbraucht.

### b) Hydrierung:

Die bei der Ozonolyse erhaltene Lösung wurde über ein Dosiergefäß in einen Hydrierreaktor, in dem eine Suspension von 1,5 g Pt in 0,5 Liter Methanol vorgelegt wurde und der mit Wasserstoff gefüllt wurde, in einer solchen Dosierrate eingespeist, dass der Peroxidgehalt im Hydrierreaktor zu Beginn und im Verlaufe der gesamten Hydrierung maximal 0,01 Mol/l betrug. Der verbrauchte Wasserstoff wurde laufend druckgeregelt ergänzt. Dabei wurde über die gesamte Hydrierperiode durch Kühlung eine Temperatur von 30°C - 33 °C, sowie durch Zugabe von methanolischer NaOH ein pH- Wert von 2 bis 3 eingehalten. Sobald der Hydrierreaktor gefüllt war, wurde laufend hydrierte Lösung über eine getauchte Fritte entnommen, um den Stand annähernd konstant zu halten. Dabei wurde die Dosierung der Peroxidlösung nicht unterbrochen. Der Peroxidgehalt wurde laufend durch jodometrische Titration kontrolliert.

Nach Beendigung der Hydrierung wurde der Hydrierreaktor über die Fritte entleert und ein polarographisch bestimmter Glyoxylsäuremethylester- Methanolhalbacetalgehalt von 5100 g = 42,5 mol (85 % der Theorie) festgestellt.

### Beispiel 3:

### a) Ozonisierung:

In einer Umlaufapparatur wie in Beispiel 1 beschrieben wurden 16 Liter einer Lösung von 3600 g DMM in Methanol in 4 Teilen zu je 4 Litern ozonisiert, bis der DMM Gehalt auf ca. 40 g/l abgesunken war. Die so erhaltene Peroxidlösung wurde tiefgekühlt bei ca. -30 °C aufbewahrt. Während der Aufbewahrung wurde weder eine exotherme Reaktion noch eine Abnahme des Peroxidgehaltes festgestellt.

In der zweiten Stufe der Ozonisierung wurde die aufbewahrte Peroxidlösung analog Beispiel 2 bis auf einen DMM Gehalt von 2-3 g/l ozonisiert. Auf diese Weise wurden insgesamt 16 Liter methanolische Peroxidlösung erhalten, es wurden dabei 3600 g DMM ozonisiert.

Die insgesamt aufgenommene Ozonmenge betrug 1252 g entsprechend 26,1 mol = 104% der Theorie.

### b) Hydrierung:

Die bei der Ozonolyse erhaltene Lösung wurde über ein Dosiergefäß in einen Hydrierreaktor, in dem eine Suspension von 1,5 g Pt in 0,5 Liter Methanol vorgelegt wurde und der mit Wasserstoff gefüllt wurde, in einer solchen Dosierrate eingespeist, dass der Peroxidgehalt im Hydrierreaktor zu Beginn und im Verlaufe der gesamten Hydrierung maximal 0,01 Mol/l betrug. Der verbrauchte Wasserstoff wurde laufend druckgeregelt ergänzt. Dabei wurde über die gesamte Hydrierperiode durch Kühlung eine Temperatur von 30°C - 33 °C, sowie durch Zugabe von methanolischer NaOH ein pH-Wert von 2 bis 3 eingehalten. Sobald der Hydrierreaktor gefüllt war, wurde kontinuierlich hydrierte Lösung über eine getauchte Fritte entnommen, um den Stand annähernd konstant zu halten. Dabei wurde die Dosierung der Peroxidlösung nicht unterbrochen. Der Peroxidgehalt wurde laufend durch jodometrische Titration kontrolliert.

Nach Beendigung der Hydrierung wurde der Hydrierreaktor über die Fritte entleert und ein polarographisch bestimmter Glyoxylsäuremethylester - Methanolhalbacetalgehalt von 48 mol (96 % der Theorie) festgestellt.

### Beispiel 4:

### a) Ozonisierung:

In einer Umlaufapparatur, wie in Beispiel 3 beschrieben, wurden 16 Liter einer Lösung von 3600 g DMM in Methanol ozonisiert, diesmal jedoch, bis der DMM Gehalt auf ca. 120 g/l abgesunken war. Die so erhaltene Peroxidlösung wurde tiefgekühlt bei ca. -30 °C aufbewahrt. Während der Aufbewahrung wurde weder eine exotherme Reaktion noch eine Abnahme des Peroxidgehaltes festgestellt.

In der zweiten Stufe der Ozonisierung wurde die aufbewahrte Peroxidlösung analog Beispiel 2 bis auf einen DMM Gehalt von 2-3 g/l ozonisiert. Auf diese Weise wurden insgesamt 16 Liter methanolische Peroxidlösung erhalten, es wurden dabei 3600 g DMM ozonisiert.

Die insgesamt aufgenommene Ozonmenge betrug 1288 g entsprechend 26,8 mol = 107% der Theorie.

### b) Hydrierung:

Die bei der Ozonolyse erhaltene Lösung wurde über ein Dosiergefäß in einen Hydrierreaktor, in dem eine Suspension von 1,5 g Pt in 0,5 Liter Methanol vorgelegt wurde und der mit Wasserstoff gefüllt wurde, in einer solchen Dosierrate eingespeist, dass der Peroxidgehalt im Hydrierreaktor zu Beginn und im Verlaufe der gesamten Hydrierung maximal 0,01 Mol/l betrug. Der verbrauchte Wasserstoff wurde laufend druckgeregelt ergänzt. Dabei wurde über die gesamte Hydrierperiode durch Kühlung eine Temperatur von 30°C - 35 °C, sowie durch Zugabe von methanolischer NaOH ein pH- Wert von 2 bis 3 eingehalten. Sobald der Hydrierreaktor gefüllt war, wurde kontinuierlich hydrierte Lösung über eine getauchte Fritte entnommen, um den Stand annähernd konstant zu halten. Dabei wurde die Dosierung der Peroxidlösung nicht unterbrochen. Der Peroxidgehalt wurde laufend durch jodometrische Titration kontrolliert.

Nach Beendigung der Hydrierung wurde der Hydrierreaktor über die Fritte entleert und ein polarographisch bestimmter Glyoxylsäuremethylester - Methanolhalbacetalgehalt von 2748 g = 47,9 mol (95,8 % der Theorie) festgestellt.

### Beispiel 5:

### a) Ozonisierung:

In einer Umlaufapparatur wie in Beispiel 3 beschrieben wurden 4 Liter einer Lösung von 900 g DMM in Methanol ozonisiert, bis der DMM Gehalt auf ca. 40 g/l abgesunken war. Dann wurden bei gleichbleibender Ozonaufgabe weitere 20 I einer Lösung von einer Konzentration von 225 g/l so dosiert, dass ein DMM Gehalt von 40 g ± 2 g/l in der Ozonolyselösung eingehalten wurde. Der Stand in der Apparatur wurde durch Entnahme der überschüssigen entstandenen Peroxidlösung konstant gehalten. Die so erhaltene Peroxidlösung wurde tiefgekühlt bei ca. -30 °C aufbewahrt. Während der Aufbewahrung wurde weder eine exotherme Reaktion noch eine Abnahme des Peroxidgehaltes festgestellt.

In der zweiten Stufe der Ozonisierung wurde die aufbewahrte Peroxidlösung analog Beispiel 2 bis auf einen DMM Gehalt von 2-3 g/l ozonisiert. Auf diese Weise wurden insgesamt 24 Liter methanolische Peroxidlösung erhalten, es wurden dabei 5400 g DMM ozonisiert.

Die insgesamt aufgenommene Ozonmenge betrug 1900 g entsprechend 39,6mol = 106% der Theorie.

### b) Die Hydrierung wurde wie in Beispiel 4 durchgeführt.

Nach Beendigung der Hydrierung wurde der Hydrierreaktor über die Fritte entleert und ein polarographisch bestimmter Glyoxylsäuremethylester - Methanolhalbacetalgehalt von 8640 g (Ausbeute 96,0% der Theorie) festgestellt.

### Beispiel 6: (Vergleichsbeispiel B: Ozonolyse Batch Hydrierung kont.)

### a) Ozonisierung:

Es wurde eine Peroxidlösung durch Ozonolyse von Naphthalin in Methanol analog Beispiel 1 hergestellt. Dazu wurden 256 g Naphthalin in 4 Liter methanolischer Lösung ozonisiert. Das anfänglich ungelöste Naphthalin löste sich im Laufe der Ozonisierung.

### b) Hydrierung:

Die bei der Ozonolyse erhaltene Lösung wurde über ein Dosiergefäß in einen Hydrierreaktor, in dem eine Suspension von 1,5 g Pt in 0,5 Liter Methanol vorgelegt wurde und der mit Wasserstoff gefüllt wurde, in einer solchen Dosierrate eingespeist, dass der Peroxidgehalt im Hydrierreaktor zu Beginn und im Verlaufe der gesamten Hydrierung maximal 0,01 Mol/l betrug. Der verbrauchte Wasserstoff wurde laufend druckgeregelt ergänzt. Dabei wurde über die gesamte Hydrierperiode durch Kühlung eine Temperatur von 30°C ± 2 °C sowie durch Zugabe von methanolischer NaOH ein pH- Wert von 2 bis 4 eingehalten. Sobald der Hydrierreaktor gefüllt war, wurde laufend hydrierte Lösung über eine getauchte Fritte entnommen, um den Stand annähernd konstant zu halten. Dabei wurde die Dosierung der Peroxidlösung nicht unterbrochen.

Nach Beendigung der Hydrierung wurde durch GC ein Gehalt an Ortho-Phthalaldehyd von 220,5 g (82,3% der Theorie) festgestellt. Die Lösung wurde zur Aufarbeitung mit H₂SO₄ auf pH 1 gestellt. Nach 4 Stunden war die Acetalisierung vollständig. Die methanolische Acetallösung wurde in überschüssige Lauge eingetropft und das Methanol gleichzeitig abdestillliert. Aus dem Reaktionsgemisch wurde das Acetal zwei mal mit MTBE extrahiert und das Lösungsmittel am Rotavapor entfernt. Es verblieb ein Rückstand von Phthalaldehyd-dimethylacetal. Das Gewicht betrug 293,3 g, entsprechend 81,5 % der Theorie.

### Beispiel 7:

### a) Ozonisierung:

In der Umlaufapparatur aus Beispiel 1 wurden 4 Liter 0,5 molare methanolische Naphthalinlösung wie in Beispiel 2 beschrieben ozonisiert. Sobald der Naphthalin Gehalt auf 2 g/l abgesunken war, wurde bei ununterbrochener O₃ Einleitung weitere 0,5 molare Naphthalinlösung eingespeist, sodass der Naphthalin Gehalt zwischen 2 und 3 g/Liter konstant gehalten wurde. Auf diese Weise wurden insgesamt 8 Liter methanolische Peroxidlösung erhalten, es wurden 512 g Naphthalin ozonisiert.

Die insgesamt aufgenommene Ozonmenge betrug 495 g entsprechend 10,31 mol = 129% der Theorie.

### b) Hydrierung:

Die Hydrierung wurde kontinuierlich wie in Beispiel 6 durchgeführt.

Nach Beendigung der Hydrierung wurde durch GC ein Gehalt an Ortho-Phthalaldehyd von 160,7 g (60 % der Theorie) festgestellt.

### Beispiel 8:

### a) Ozonisierung:

In einer Umlaufapparatur wie in Beispiel 3 beschrieben wurden 8 Liter einer Lösung von 512 g Naphthalin in Methanol ozonisiert bis der Naphthalin Gehalt auf ca. 20 g/l abgesunken war. Die so erhaltene Peroxidlösung wurde tiefgekühlt bei ca. -30 °C aufbewahrt. Während der Aufbewahrung wurde weder eine exotherme Reaktion noch eine Abnahme des Peroxidgehaltes festgestellt.

In der zweiten Stufe der Ozonisierung wurde die aufbewahrte Peroxidlösung analog Beispiel 2 bis auf einen Naphthalin Gehalt von 2-3 g/l ozonisiert. Auf diese Weise wurden insgesamt 8 Liter methanolische Peroxidlösung erhalten.

Die insgesamt aufgenommene Ozonmenge betrug 458 g entsprechend 9,54 mol = 119 % der Theorie.

### b) Hydrierung:

Die Hydrierung wurde kontinuierlich wie in Beispiel 6 durchgeführt.

Nach Beendigung der Hydrierung wurde durch GC ein Gehalt an Ortho-Phthalaldehyd von 417,9 g (78 % der Theorie) festgestellt.

### Beispiel 9: (Vergleichsbeispiel C)

4 Liter einer Lösung von 600g Methylmethacrylat in Methanol mit Zusatz von 0,1 g Hydrochinon zur Verhinderung einer Polymerisation wurden wie in Beispiel 2 bis zu einem MMA Gehalt von 1 g/l ozonisiert und danach mit einem Lindlar Katalysator 5%Pd/Pb auf CaCO₃ bei pH 5 hydriert. Ein Teil des eingesetzten Methylmethacrylat wurde bei der Ozonolyse durch das O₂ -Abgas ausgetragen. Der Ozonverbrauch betrug mit 266 g nur 92% der Theorie. Nach der Hydrierung wurde der in der Lösung enthaltene Brenztraubensäuremethylester (Methylpyruvat) mit 528 g entsprechend 86,3% d. Th. bezogen auf eingesetztes Methylmethacrylat bestimmt.

### Beispiel 10:

In der Umlaufapparatur wurde ein minimales Volumen von 1,5 I Methanol mit einer Konzentration von 2 g/l Methylmethacrylat vorgelegt und gleichzeitig die Ozonisierung und die Dosierung von 4 Litern einer MMA Lösung der Konzentation 150 g/l begonnen, sodass der Methylmethacrylat (MMA) Gehalt etwa im Bereich 1g/l ± 1g/l konstant blieb. Insgesamt wurden 603 g Methylmethacrylat ozonisiert, der Ozonverbrauch betrug 283 (98% d.Th)

Die Hydrierung wurde analog Beispiel 9 durchgeführt. In der Hydrierlösung wurden 567,8 g Methylpyruvat gefunden. (92,8% d.Th.)

### Beispiel 11:

In der Umlaufapparatur wurde ein minimales Volumen von 1,5 I Methanol mit einer Konzentration von 20 g/l Methylmethacrylat vorgelegt und gleichzeitig die Ozonisierung und die Dosierung von 8 Litern einer MMA Lösung der Konzentration 150 g/l begonnen, sodass der MMA Gehalt etwa im Bereich 20g/l ± 1g/l konstant blieb. Die in der Ozonolyse entstandene Peroxid lösung wurde bei -30 °C für die zweite Stufe der Ozonolyse aufbewahrt. In der zweiten Stufe der Ozonolyse wurden 1,5 I Peroxidlösung aus Stufe 1 vorgelegt und bis zu einer Konzentration von ca. 1 g/l Methylmethacrylat ozonisiert. Dann wurde bei weiter laufender Ozonolyse die Peroxidlösung aus Stufe 1 so dosiert, dass die Konzentration an Methylmethacrylat bei 1g/l ± 1 g konstant gehalten wurde. Die Peroxidlösung wurde der Apparatur kontinuierlich so entnommen, dass der Stand an Lösung in der Apparatur etwa konstant blieb.

Insgesamt wurden 1230 g Methylmethacrylat ozonisiert, der Ozonverbrauch betrug 562 (95% d.Th.)

Die Hydrierung wurde analog Beispiel 9 durchgeführt. In der Hydrierlösung wurden 1148 g Methylpyruvat gefunden. (91,5% d.Th. bezogen auf eingesetztes Methylmethacrylat)

### Beispiel 12: (Vergleichsbeispiel D)

Methylmethacrylat wurde wie in Beispiel 9 ozonisiert und kontinuierlich hydriert, als Katalysator wurde jedoch Platinschwamm, erhalten durch Hydrierung von 3 g PtO2 , eingesetzt. Die Hydrierung erfolgte wie in Beispiel 9 bei pH 5. Es wurden inklusive der Spüllösungen 4,6 Liter einer methanolischen Lösung erhalten, die 549 g Methyllactat enthielt. (88,1 % d. Th. bezogen auf Methylmethacrylat und 95,7% bezogen auf eingesetztes Ozon)

### Beispiel 13:

4 Liter einer Lösung von 600 g Methylmethacrylat wurden analog Beispiel 10 ozonisiert und wie in Beispiel 12 hydriert. In der Hydrierlösung wurden 580 g Methyllactat ( 93% d.Th. bezogen auf eingesetztes Methylmethacrylat) gefunden.

### Beispiel 14: (Vergleichsbeispiel E)

4 Liter einer Lösung von 440 g Cycloocten in Methanol wurden wie in Beispiel 1 ozonisiert. Sofort mit Beginn der Ozonolyse zeigte sich starke Nebelbildung im Abgas. Die Nebelbildung war weitgehend unabhängig von der Ozonkonzentration, trat aber bei Verwendung von reinem Sauerstoff ohne Ozon nicht auf. Die Ozonolyse wurde aus Sicherheitsgründen unterbrochen und die Apparatur entleert und gereinigt.

### Beispiel 15:

Es wurden 4 Liter einer Lösung von 440 g Cycloocten (4 mol) in Methanol hergestellt. Es wurden wie in Beispiel 10 beschrieben, 1,5 Liter Methanol vorgelegt und auf einmal 30ml Cyclooctenlösung dosiert, sodass ca. 2g/l Cycloocten erhalten wurden. Die Dosierung wurde angehalten und dann die Ozonisierung mit gleichzeitiger Dosierung von Cyclooctenlösung begonnen. Es wurde soviel Cycloocten dosiert, dass in der Ozonolyselösung eine Cyclooctenkonzentration von 2g/l ± 1g/l aufrecht erhalten wurde. Nebelbildung im Abgas wurde bei dieser Konzentration nicht beobachtet. Der Ozonverbrauch betrug 92 g (96 d.Th.)

Die Peroxidlösung wurde wie in Beispiel 2 kontinuierlich hydriert. Es wurden 5,2 Liter einer methanolischen Lösung mit 522 g Octandial (92 % d.Th. bezogen auf eingesetztes Cycloocten) erhalten. Die Lösung wurde mit Schwefelsäure auf pH 1 gestellt, über Nacht bei Raumtemperatur stehen gelassen und mit NaOH auf pH 10 gestellt. Anschließend wurde 1 Liter Wasser zugesetzt und das Methanol bei einer Wasserbadtemperatur von 100 °C mit einem Rotavapor entfernt. Vom zweiphasigen Rückstand wurde die organische Phase abgetrennt, die wässrige Phase 1 mal mit 100 ml MTBE extrahiert, die organischen Phase vereinigt, mit Na2SO4 getrocknet und im Vakuum fraktioniert. Es wurden 855 g 1,1,8,8-Tetramethoxyoctan vom Kp₃₀ = 147 - 149°C erhalten (91% d.Th.)

### Beispiel 16:

4 Liter einer Lösung von 417 g (4 mol) Vinylpyridin in Methanol wurden analog Beispiel 15 ozonisiert, wobei durch ständiges Dosieren von Vinylpyridinlösung in der Ozonolyse eine Konzentration von ca. 2 g Vinylpyridin pro Liter aufrecht erhalten wurde. Bei dieser Konzentration ist die Ozonaufnahme noch quantitativ. Die Ozonaufnahme beträgt 196 g (102% d.Th.) Die Hydrierung wurde Batch ohne pH Regelung bei 20 °C über 4 g 10% Pd Katalysator auf Aktivkohle wie in Beispiel 1 beschrieben durchgeführt. Die Produktlösung wurde gaschromatographisch analysiert und die Ausbeute an Pyridinaldehyd mit 347 g (81 % d.Th.) bestimmt.

### Beispiel 17:

8 Liter einer Lösung von 834 g Vinylpyridin in Methanol wurden in einer ersten Stufe wie in Beispiel 11 kontinuierlich bei einer Vinylpyridin Konzentration von 20 g/l , in einer zweiten Stufe bei einer Vinylpyridin Konzentration von 2 g/l ozonisiert. Die Ozonaufnahme beträgt 380 g (98,9% d.Th.) Die erhaltene Peroxidlösung wurde kontinuierlich ohne pH Kontrolle bei maximal 20 °C über 4 g 10% Pd Katalysator auf Aktivkohle hydriert. Die Produktlösung wurde gaschromatographisch analysiert und die Ausbeute an Pyridinaldehyd mit 720 g ( 84 % d.Th.) bestimmt.

### Beispiel 18:

4 Liter einer Lösung von 417 g (4 mol) Vinylpyridin in Methanol wurden analog Beispiel 16 ozonisiert und mit 4 g 10% Pd Katalysator auf Aktivkohle bei mindestens 40 ± 2 °C batch ohne Laugezusatz hydriert. Der Ozonverbrauch betrug 196 g (102% d.Th.) Die ozonisierte Lösung wurde so in die Hydrierapparatur eingespeist, dass ein Peroxidgehalt von 10 mmol nicht überschritten wurde. Die dabei gebildete Pyridinaldehydkonzentration betrug weniger als 1%. Die hydrierte Produktlösung wurde langsam in überschüssige wässrige Natronlauge eingetragen, und das Methanol gleichzeitig abdestilliert. In diesem Schritt wurde in der Hydrierung erhaltenes Pyridinaldehyd zu Hydroxymethylpyridin (HMP) und der gleichzeitig entstandene Formaldehyd zu Formiat und Methanol disproportioniert. Aus dem erhaltenen alkalischen Reaktionsgemisch wurde das HMP 10 mal mit MTBE extrahiert, von den organischen Phasen das MTBE abdestilliert und der Rückstand bei 80 mbar und 143 °C fraktioniert. Es wurden 332,1 g (76 % d.Th. ) reines HMP als farblose Flüssigkeit erhalten.

### Beispiel 19:

Es wurden 12 Liter einer Lösung von 1250 Vinylpyridin (12 mol) in Methanol hergestellt und eine 1 molare Lösung von Vinylpyridin in Methanol wurd analog Beispiel 3 ozonisiert, wobei durch Zudosieren eine Konzentration von 20 g Vinylpyridin pro Liter in der Ozonolyse aufrecht erhalten wurde. Insgesamt wurden 12 I Lösung und damit 12 mol Vinylpyridin eingespeist. Das eingeleitete Ozon wurde quantitativ aufgenommen. Die erhaltene Peroxidlösung wurde tiefgekühlt bei -30 °C gelagert und für eine weitere kontinuierliche Ozonolyse bei einem Vinylpyridin-Gehalt von ca. 2 g/l verwendet. Der gesamte Ozonverbrauch lag mit 570 g oder 98,9 % d.Th. etwas niedriger als in Beispiel 18. Die erhaltene Peroxidlösung wurde analog Beispiel 17 hydriert. Nach Aufarbeitung analog Beispiel 18 wurden 1024 (78,1% d.Th.) Hydroxymethylpyridin erhalten.

### Beispiel 20 (Vergleichsversuch F):

4 Liter einer Lösung von 220 g Cycloocten in Methanol wurden wie in Beispiel 1 ozonisiert. Sofort mit Beginn der Ozonolyse zeigte sich starke Nebelbildung im Abgas. Die Nebelbildung war weitgehend unabhängig von der Ozonkonzentration, trat aber bei Verwendung von reinem Sauerstoff ohne Ozon nicht auf. Die Ozonolyse wurde wie in Beispiel 14 aus Sicherheitsgründen unterbrochen und die Apparatur entleert und gereinigt.

### Beispiel 21:

Es wurden 4 Liter einer Lösung von 220 g Cycloocten (4,07 mol) in Methanol hergestellt. Die Ozonisierung wurde wie in Beispiel 15 beschrieben durchgeführt, wobei in der Ozonolyselösung eine maximale Konzentration von 2 g Cyclooctadien eingehalten wurde. Nebelbildung im Abgas wurde bei dieser Konzentration nicht beobachtet. Der Ozonverbrauch betrug 190 g (97,3 d.Th)

Die Peroxidlösung wurde wie in Beispiel 15 kontinuierlich hydriert. Es wurden 5,2 Liter einer methanolischen Lösung erhalten. Die Gesamtausbeute wurde durch Oximtitration bestimmt und ergab 330 g Succindialdehyd (94,3% d.Th.)

Charakterisierung: Die Lösung wurde mit der molaren Menge Trimethylorthoformiat versetzt, mit Schwefelsäure auf pH 1 gestellt, über Nacht bei Raumtemperatur stehen gelassen und mit NaOH auf pH 10 gestellt. Anschließend wurde 1 Liter Wasser zugesetzt und das Methanol bei einer Wasserbadtemperatur von 100 °C mit einem Rotavapor entfernt. Vom zweiphasigen Rückstand wurde die organische Phase abgetrennt, die wässrige Phase 3 mal mit 100 ml MTBE extrahiert, die organischen Phase vereinigt, mit Na₂SO₄ getrocknet und im Vakuum bei 15 mbar fraktioniert.

Es wurden 486,5 g 1,1,4,4- Tetramethoxybutan vom Kp₁₅ = 86 - 88 °C erhalten (90,5% d.Th.)

### Beispiel 22 (Vergleich G):

In einem Kleinversuch wurde eine 1 molare Lösung von Pinen in Methanol hergestellt und ozonisiert. Es trat sofort zu Beginn der Ozonolyse Nebelbildung im Abgas auf und der Versuch wurde unterbrochen. Die Ozonisierungslösung wurde soweit verdünnt, bis keine Nebelbildung mehr zu beobachten war. Die dabei eingestellt Konzentration an Pinen betrug ca. 28 g/l (=0,21 mol/l).

### Beispiel 23:

4 Liter einer Lösung von 400 g Pinen in Methanol wurden wie in Beispiel 15 mit 130 g Ozon (92,3% d.Th.) ozonisiert und kontinuierlich bei 30 °C und pH 4,5 hydriert. Der Nopinongehalt in der Hydrierlösung wurde gaschromatographisch zu 363 g (89,6% d.Th. bezogen auf eingesetztes Pinen) bestimmt. Die Hydrierlösung wurde mit 1 I Wasser versetzt, mit H₂SO₄ auf pH 5 gestellt, und das Methanol über eine Kolonne abdestiliert. Das erhaltene zweiphasige Gemisch enthielt geringe Anteile eines Feststoffs mit einem exothermen Potential von ca. 1200 J/g. Das Gemisch wurde daher mit Wasserdampf destilliert, bis das Destillat einphasig wurde und der Nopinongehalt im Destillat unter 2 g/l abgesunken war. Das Destillat wurde 2 mal mit MTBE extrahiert und die vereinigten organischen Phasen fraktioniert. Es wurden 356 g ( 87,6% d.Th. bezogen auf eingesetztes Pinen) reines Nopinon erhalten.

### Beispiel 24:

4 Liter einer Lösung von 900 g Butendiol(1,4) dibutyrat (3,94 mol) in Methanol wurden wie in Beispiel 15 mit 191 g Ozon (101 % d.Th.) ozonisiert und kontinuierlich bei 30 °C und pH 3,5 hydriert. In der Hydrierlösung wurde der Gehalt an Butyroxyacetaldehyd zu 933,8 g (91,0% d.Th. ) bestimmt.

### Beispiel 25: (Vergleichsbeispiel H)

4 Liter einer methanolischen Lösung von 470g Sulfolen wurden wie in Beispiel 1 bis zu einem Sulfolen Gehalt von < 2 g/l ozonisiert und danach wie in Beispiel 2 mit 2 g Adams Katalysator bei pH 3,5 hydriert. Der Ozonverbrauch betrug mit 189 g 99% d.Th. Nach Abtrennen des Katalysators ergab die Oximtitration einen Gehalt von 568 g 3-Thiaglutaraldehyd-3,3-dioxid (95,1 % der Theorie).

### Beispiel 26:

8 Liter einer methanolischen Lösung von 940g Sulfolen wurden wie in Beispiel 15 bis zu einem Sulfolen Gehalt von < 2 g/l ozonisiert und danach kontinuierlich wie im gleichen Beispiel mit 2 g Adams Katalysator bei pH 3,5 hydriert. Der Ozonverbrauch betrug mit 388 g (101,6 % d.Th.) und liegt damit etwas höher als in Beispiel 25.

Nach Abtrennen des Katalysators ergibt die Oximtitration einen Gehalt von 1140 g 3-Thiaglutaraldehyd-3,3-dioxid (94,8 % der Theorie).

### Beispiel 27: (Vergleichsbeispiel I)

4 Liter einer Lösung von 300 g 2,5-Dihydrofuran (4,28 mol) in Methanol wurden wie in Beispiel 1 mit 163 g Ozon (79 % d.Th.) ozonisiert und kontinuierlich bei 30 °C und pH 3,5 hydriert. Ein Teil des Dihydrofurans wurde während der Ozonolyse mit dem Abgasstrom ausgetragen.

In der Hydrierlösung wurde der Gehalt an 3-Oxaglutaraldehyd durch Oximtitration zu 321 g (73,5% d.Th. ) bestimmt.

### Beispiel 28:

8 Liter einer Lösung von 600 g 2,5-Dihydrofuran (4,28 mol) in Methanol wurden wie in Beispiel 2 mit 398 g Ozon (96,9 % d.Th.) ozonisiert und kontinuierlich bei 30 °C und pH 3,5 hydriert. Der Gehalt an Dihydrofuran im Abgas lag unter 2% der Einsatzmenge.

In der Hydrierlösung wurde der Gehalt an 3-Oxaglutaraldehyd durch Oximtitration zu 833 g (95,3% d.Th. ) bestimmt.

### Beispiel 29: Vergleichsversuch PDC Batch in Umlaufapparatur:

### Ozonisierung:

In eine kontinuierliche Umlaufapparatur, bestehend aus einer Absorptionskolonne, Trenngefäß, Umwälzpumpe und externem Wärmetauscher werden 4 kg einer wässrigen Lösung von 240g Chinolin ( 6 Gew%, entsprechend 0,464 mol/kg) und 330 g konz. Schwefelsäure vorgelegt. Die Temperatur wird durch Kühlung über den externen Wärmetauscher auf 0 bis +3 °C gekühlt. Die Kreislaufmenge beträgt ca. 220 I/h.

Die Lösung wird in der Absorptionskolonne mit 2500 NI/h Ozon/Sauerstoffstrom mit einem Ozongehalt von 96 g/Nm3 in Kontakt gebracht und reagiert mit dem enthaltenen Ozon. Das Ozon wird nicht quantitativ aufgenommen. Die Konzentration an nicht reagiertem Ozon im Abgas betrug 26 g/Nm3 zu Beginn des Batches (entsprechend 27% unreagiertem Ozon) und 61 g/Nm3 zu Ende des Batches (entsprechend 63,5% unreagiertem Ozon). Im Trenngefäß am Fuß der Absorptionskolonne trennt sich das Gemisch in eine Flüssigphase und eine Gasphase.

Nach beendeter Ozonolyse beträgt der Chinolingehalt ca. 0,7 g/l entsprechend 1,1% der Ausgangsmenge.

Die aufgenommene Ozonmenge wurde bestimmt und betrug insgesamt ca. 194g entsprechend 111 % der Theorie.

### Oxidation:

Die bei der Ozonolyse erhaltene Lösung wird bei 2 bis 5 °C mit 210 g auf + 5 °C gekühltem 30% H2O2 versetzt. Durch die Reaktionswärme steigt die Temperatur langsam an und wird durch Kühlung unter 20 °C gehalten. Während der Reaktion beginnt bereits PDC zu kristallisieren. Nach 8 Sunden ist die Reaktion beendet.

Der pH Wert wird auf 1,5 gestellt und das Reaktionsgemisch wird auf 0 °C gekühlt.

Die ausgefallene PDC wird über eine Nutsche filtriert, mit Methanol gewaschen und im Vakuum bei 40 °C bis zu Gewichtskonstanz getrocknet. Die Ausbeute an kristallisierter PDC beträgt 217 g, entsprechend 71% der Theorie, in der Mutterlauge sind weitere 30 g enthalten.

Die Mutterlauge wird auf ein Drittel ihres Volumens im Vakuum eingedampft. Nach Kühlung auf 0 °C, Filtration und Trocknung werden weitere 24 g PDC (8% d.Th.) erhalten. Die Ausbeute an isolierter PDC ist somit 241 g(79% d.Th) berechnet auf Chinolin. Gesamtausbeute 247 g ( 81 %)

### Beispiel 30: PDC kontinuierlich in zwei Blasensäulen mit Ozon-split

Es wurde in 2 Blasensäulen (von 2000 mm Länge und 100 mm Durchmesser (Gesamtvolumen 15,7 Liter) )ozonisiert. Die Apparatur war mit einer Dosierpumpe zur Dosierung von Chinolinlösung in die erste Blasensäule und einer weiteren Dosierpumpe zum Dosieren bereits ozonisierter Lösung aus der ersten Blasensäule in die zweite Blasensäule ausgerüstet. Jede Blasensäule war mit einer Vorrichtung zum Dosieren von frischem Ozon versehen.

Vorbereitung: Beide Blasensäulen wurden mit je 12 kg einer wässrig schwefelsauren Lösung von Chinolin (Konzentration wie in Beispiel 29) befüllt. Dann wurde in die erste Blasensäule Ozon bis zu einem Chinolingehalt von 15 g/l, in die zweite Blasensäule bis zu einen Chinolingehalt von 1 g/l eingeleitet. Die Gasmenge betrug jeweils 10 Nm3/h, der Druck 5,3 bar abs und der Ozongehalt 110 g/Nm3.

Kontinuierlicher Betrieb: In die zweite Blasensäule (mit der niedrigen Chinolinkonzentration) wurden 3,5 Nm3/h Ozongas, in die erste Blasensäule 10 Nm3/h Ozongas eingeleitet. Die Chinolinkonzentration wurde durch Dosieren von Reaktionslösung aus der ersten in die zweite Blasensäule zwischen 0,8 und 1 g/l gehalten, die aus der ersten Säule entnommene Reaktionslösung wurde durch das gleiche Volumen frischer Chinolin Lösung ersetzt, durch Entnahme einer entsprechenden Menge fertig ozonisierter Lösung aus der zweiten Blasensäule wurde dort das Flüssigkeitvolumen ebenfalls konstant gehalten. Nach einigen Stunden stellte sich in der ersten Blasensäule eine gleichbleibende Konzentration von 12 g Chinolin / L ein. In der ersten Blasensäule reagierten 97% des eingesetzten Ozons, in der zweiten Blasensäule 95% des eingesetzten Ozons. Es wurde keine Ausfällung von PDC beobachtet. Die Verhältnisse der Chinolinkonzentrationen in den Blasensäulen entsprachen ziemlich genau den Verhältnissen der zur Reaktion gebrachten Ozonmengen.

Der Ozonverbrauch wurde gemessen und betrug ca. 107% der Th, berechnet auf Chinolin. Der Ozonverbrauch war damit nur gering höher als der Verbrauch bei Batch Fahrweise unter Druck.

Aus dem kontinuierlichen Betrieb wurden zu Beginn und am Ende jeweils 1 kg Probe gezogen, diese Probe wie in Beispiel 3 mit H2O2 oxidiert und aufgearbeitet.

Die isolierte PDC Ausbeute betrug zu Beginn des kontinuierlichen Versuchs 58,8 g/kg Probe (77%) und 6,1 g/kg Probe (8% ), entsprechend einer isolierten Gesamtausbeute von 85 %, am Ende 58,4 g/kg Probe (76,5%) und 6 g/kg Probe (7,8%), entsprechend einer isolierten Gesamtausbeute von 84,3 %.

### Beispiel 31: Vergleichsversuch: PDC kontinuierlich in der Umlaufapparatur

Eine Umlaufapparatur wie in Beispiel 29 wurde kontinuierlich betrieben:

Es werden 4 kg einer wässrigen schwefelsauren Lösung von Chinolin (Zusammensetzung: 6 Gew% Chinolin, 9,1 Gew% Schwefelsäure , Rest Wasser wie in Beispiel 29) mit 2500 Nl O2/O3 mit 100 gO3/Nm3 batch ozonisiert, bis der Chinolingehalt auf 0,9 g/l abgesunken ist. Dann wird mit gleicher Gasmenge und Ozonkonzentration weiter ozonisiert und die Chinolinkonzentration durch Zudosierung von wässrig schwefelsaurer Chinolinlösung zwischen 0,7 g/l und 0,9 g/l gehalten. Das Abgas enthielt zu Beginn der kontinuierlichen Fahrweise 63 g O3/Nm3, bei Ende des Versuchs 59 gO3/Nm3. Der Ozonverbrauch wurde gemessen und betrug 230% der Th., berechnet auf Chinolin, was auf massive Nebenreaktionen und Weiterreaktionen der Spaltprodukte schließen lässt. Schließlich begann PDC aus der Lösung auszufallen, welche die Absorptionskolonne verstopfte.

Aus dem kontinuierlichen Betrieb wurden zu Beginn und am Ende vor dem PDC Ausfall jeweils 1 kg Probe gezogen und diese Probe wie in Beispiel 29 mit H2O2 oxidiert.

Die isolierte PDC Ausbeute betrug 51,3 g/kg Probe und 5,3 g/kg Probe zu Beginn, entsprechend einer isolierten Gesamtausbeute von 74 %, am Ende 47,2 g/kg Probe und 5,1 g/kg Probe, entsprechend einer isolierten Gesamtausbeute von 68,5 %.

## Patentansprüche

1. Verfahren zur Herstellung von Mono- oder Biscarbonyl- oder Hydroxylverbindungen durch Ozonisierung von ungesättigten organischen Kohlenstoffverbindungen, die eine oder mehrere durch Ozon spaltbare olefinischen oder aromatischen Doppelbindungen im Molekül aufweisen und anschließender Aufarbeitung der Ozonisierungsprodukte, **dadurch gekennzeichnet, dass** ungesättigte organische Kohlenstoffverbindungen, die eine oder mehrere durch Ozon spaltbare olefinische oder aromatische Doppelbindung im Molekül aufweisen,
a) in einem organischen Lösungsmittel oder in einer wässrigen Lösung in 1 bis 2 Schritten kontinuierlich in einer Vorrichtung bestehend aus zwei Absorptionsapparaturen, Vorrichtungen zur Abfuhr der Reaktionswärme und Vorrichtungen zum Trennen von Gas- und Flüssigphase, mit gegenläufigen Eduktströmen mit Ozon in stöchiometrischen Mengen oder im Überschuss umgesetzt und
b) die dabei entstehenden Peroxide in Abhängigkeit von den Reaktionsparametern aus Schritt a) entweder durch kontinuierliche oder diskontinuierliche Hydrierung, Oxidation oder Erwärmen in die entsprechenden Mono- oder Biscarbonyl- oder Hydroxylverbindungen überführt werden,
wobei in Schritt a)
das Edukt mit einer vom eingesetzten Edukt und den Reaktionsbedingungen abhängigen Ausgangskonzentration in die erste Absorptionsapparatur eingespeist, der Ozon-führende O₂-Strom mit einer von der Reaktivität des Eduktes abhängigen Ozonkonzentration hingegen in die zweiten Absorptionsapparatur eingebracht wird, sodass in der 1. Absorptionsapparatur das eingesetzte Edukt mit dem Ozonstrom in Kontakt gebracht wird, der nach Durchlauf der 2. Absorptionsapparatur in die 1. Absorp-tionsapparatur eingespeist wird, wodurch in der 1. Absorptionsapparatur ein Ozonunterschuss vorliegt, anschließend das Reaktionsgemisch, nach der Reaktion des in die 1. Absorptionsapparatur eingespeisten Ozons mit dem entsprechend eingebrachten Edukts, aus der 1. Absorptionsapparatur austritt, in eine Gasphase und eine flüssige Phase getrennt wird, worauf die flüssige Phase, die noch nicht umgesetztes Edukt, Lösungsmittel und das entsprechende Ozonolyseprodukt enthält, in die 2. Absorptionsapparatur eingespeist wird, in die der Ozon-führende O₂-Strom, mit der gewünschten Ausgangskonzentration an Ozon eingebracht wird, wodurch in dieser Apparatur ein Ozonüberschuss vorliegt, worauf nach beendeter Umsetzung das Reaktionsgemisch nach Austritt aus der 2. Absorptionskolonne wiederum in eine Gasphase und eine flüssige Phase getrennt wird, worauf die flüssige Phase, die jetzt nur mehr das entsprechende Ozonolyseprodukt in dem eingesetzten Lösungsmittel enthält, sodann der Aufarbeitungsstufe b) zugeführt wird und der in der Gasphase enthaltene geringe Prozentsatz an Ozon gegebenenfalls in die 1. Absorptionsapparatur, zur weiteren Umsetzung neu eingespeisten Edukts, eingebracht wird.

2. Verfahren nach Anspruch 1, **dadurch gekennzeichnet, dass** die Ozonmenge in Abhängigkeit vom eingesetzten Edukt so gewählt wird, dass sie für reaktive Substanzen einem beinahe stöchiometrischem Ozonverbrauch bis zu etwa 107% der stöchiometrischen Menge und für wenig reaktive Substanzen einem Ozonverbrauch von etwa 107 bis 140%, bevorzugt bis 120%, der stöchiometrischen Menge bezogen auf das Edukt entspricht.

3. Verfahren nach Anspruch 1, **dadurch gekennzeichnet, dass** die Ausgangskonzentration des Eduktes in Abhängigkeit vom eingesetzten Edukt und den Reaktionsbedingungen zwischen 1 und 3 mol/l, bezogen auf die Doppelbindungen, liegt.

4. Verfahren nach Anspruch 1, **dadurch gekennzeichnet, dass** bevorzugt im Vergleich zum Lösungsmittel oder zum gebildeten Ozonolyseprodukt schnell reagierende Edukte eingesetzt werden.

5. Verfahren nach Anspruch 1, **dadurch gekennzeichnet, dass**
der Eduktstrom in die 1. Absorptionsapparatur eingeleitet und der Ozon-führende O₂₋Strom gleich in die 1. Absorptionsapparatur eingebracht wird, wobei ein Teil des Stromes für die 2. Apparatur abgezweigt und in diese eingespeist wird, sodass ein Ozon-split durchgeführt wird, wodurch in der 1. Absorptionsapparatur wiederum ein Ozonunterschuss vorliegt,
worauf nach beendeter Umsetzung in der 1. Absorptionsapparatur das Reaktionsgemisch in eine Gasphase und eine flüssige Phase getrennt wird,
die flüssige Phase, die hauptsächlich das entsprechende Ozonolyseprodukt in dem eingesetzten Lösungsmittel und restliches, noch nicht umgesetztes Edukt enthält in die 2. Absorptionsapparatur eingebracht wird, wo sie mit dem abgezweigten Ozonstrom in Kontakt gebracht wird, wodurch in der 2. Absorptionsapparatur ein Ozonüberschuss vorliegt,
worauf nach beendeter Umsetzung das Reaktionsgemisch wiederum in eine Gasphase und eine flüssige Phase getrennt wird
und die flüssige Phase, die jetzt nur mehr das entsprechende Ozonolyseprodukt in dem eingesetzten Lösungsmittel enthält, der Aufarbeitungsphase (Schritt b) zugeführt wird.

6. Verfahren nach Anspruch 5, **dadurch gekennzeichnet, dass** die Aufteilung des Ozon-führende O₂-Stromes in einem Verhältnis von 1. Absorptionsapparatur zu 2. Absorptionsapparatur von 50 : 50 bis 90 : 10, erfolgt, wobei der in Absorptionsapparatur 1 und 2 eingespeiste O₂-Strom 4-10% Ozon enthält.

7. Verfahren nach Anspruch 5 und 6, **dadurch gekennzeichnet, dass** die Eduktkonzentration nach Verlassen der 1. Absorptionsapparatur von dem Aufteilungsverhältnis des Ozonstromes abhängt und bei einer Aufteilung von 90:10 bevorzugt 0,1 mol/l bis 0,5 mol/l und bei einer Aufteilung von 50:50 bevorzugt 0,9 bis 2 mol/l beträgt.

8. Verfahren nach Anspruch 1-7, **dadurch gekennzeichnet, dass** als Absorptionsapparatur Apparaturen verwendet werden, die einen Gas-Flüssig-Austausch bewerkstelligen.

9. Verfahren nach Anspruch 8, **dadurch gekennzeichnet, dass** als Absorptionsapparaturen Absorptionskolonnen, Blasensäulen, gerührte Reaktoren, Rührkessel, Mischer oder Schleifenreaktoren eingesetzt werden.

10. Verfahren nach Anspruch 1-9, **dadurch gekennzeichnet, dass** für die Ozonolyse im wässrigen System Blasensäulen als Absorptionsapparaturen verwendet werden.

11. Verfahren nach Anspruch 10, **dadurch gekennzeichnet, dass** das bei der Ozonolyse im wässrigen System Blasensäulen als Absorptionsapparaturen verwendet werden und ein Ozon-split gemäß Anspruch 6 durchgeführt wird.

12. Verfahren nach Anspruch 1, **dadurch gekennzeichnet, dass** die in Schritt a) erhaltenen Peroxide in Schritt b) durch kontinuierliche oder diskontinuierliche Hydrierung in die entsprechenden Mono- oder Biscarbonyl- oder Hydroxylverbindungen überführt werden, in dem die aus der Ozonolysestufe erhaltene peroxidhaltige Ozonolyseproduktlösung und ein Wasserstoffstrom in eine einen ausreichenden Massentransfer von Wasserstoff in die flüssige Phase gewährleistende Hydrierapparatur eingeleitet wird, in der ein Lösungsmittel und ein Hydrierkatalysator vorgelegt werden, gegebenenfalls unter gleichzeitig Zudosierung eines basischen Zusatzes zur Regelung des pH-Wertes, worauf das Volumen der Reaktorlösung durch standgeregelten Austrag über die Filtrationseinheit konstant gehalten wird, wodurch der Peroxidgehalt, der ausgetragenen Lösung, der unter 0,01 mo/l liegt, laufend kontrolliert wird.

13. Verfahren nach Anspruch 12, **dadurch gekennzeichnet, dass** als Hydrierapparaturen Rührkessel, Schleifenreaktoren, gerührte oder ungerührte Blasensäulen oder Festbettreaktoren eingesetzt werden.

14. Verfahren nach Anspruch 1-13, **dadurch gekennzeichnet, dass** Mono- oder Biscarbonyl- oder Hydroxylverbindungen der allgemeinen Formel I 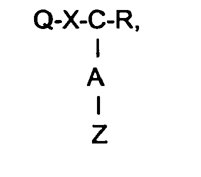 worin
Z entweder OH oder O bedeutet und A für Z gleich OH eine Einfachbindung und für Z gleich O eine Doppelbindung darstellt
Q Wasserstoff oder die Reste, 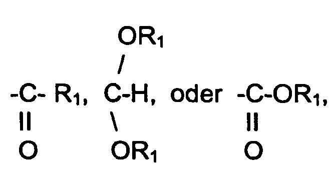 oder -OR₁
bezeichnet, wobei R₁ H bedeutet oder für einen Esterteil steht, der sich von chiralen
oder nicht chiralen, primären, sekundären oder tertiären Alkoholen ableitet,
X einen geradkettigen oder verzweigten ein- oder zweiwertigen, aliphatischen Alkyl- oder Alkylenrest mit 1 bis 50 C-Atomen, wobei dieser Alkyl- oder Alkylenrest durch eine oder mehrere Gruppen, die unter den Reaktionsbedingungen inert sind, substituiert sein kann; einen gegebenenfalls substituierten, geradkettigen oder verzweigten aliphatischen Alkyl- oder Alkylenrest mit 2 bis 50 C-Atomen, wobei eine oder mehrere der - CH₂-Gruppen der Alkyl- bzw. Alkylenkette durch ein Sauerstoffatom, ein Stickstoffatom, ein Schwefelatom oder eine -SO₂-Gruppe ersetzt ist; einen Rest der Formel -(CH₂)ₘ-O-CO-(CH₂)ₚ, wobei m eine ganze Zahl von 1 bis 4 und p eine ganze Zahl von 1 bis 6 sein kann; einen Phenyl- oder Phenylenrest, wobei dieser Phenyl- oder Phenylenrest durch eine oder mehrere Gruppen, die unter den Reaktionsbedingungen inert sind, substituiert sein kann; einen ein- oder zweiwertigen Alkylarylen- oder Alkylenarylenrest mit 7 bis 50 C-Atomen, wobei diese Reste durch eine oder mehrere Gruppen, die unter den Reaktionsbedingungen inert sind, substituiert sein können; einen gegebenenfalls substituierten Heterocyclus mit einem oder zwei Heteroatomen im Ring oder eine Einfachbindung zwischen zwei benachbarten C-Atomen bedeutet, und
R Wasserstoff, einen C₁ bis C₂₀-Alkylrest, -OR₁ oder den
Rest 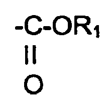 bezeichnet,
oder X und R gemeinsam einen mono- oder bicyclischen Rest mit 4 bis 20 C-Atomen bilden, der ein- oder mehrfach durch unter den Reaktionsbedingungen inerte Gruppen substituiert sein kann,
hergestellt werden.

15. Verfahren nach Anspruch 1-14, **dadurch gekennzeichnet, dass** als ungesättigte organische Kohlenstoffverbindungen, die eine oder mehrere durch Ozon spaltbare olefinische oder aromatische Doppelbindung im Molekül aufweisen,
Verbindung der allgemeinen Formel II 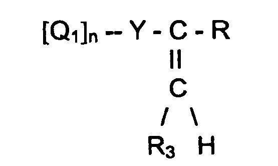 worin n 0 oder 1 ist, Q₁ Wasserstoff oder die Reste 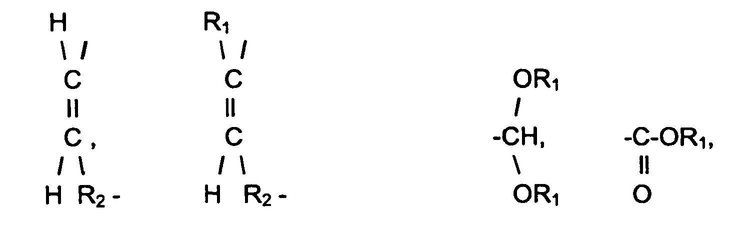 bezeichnet, wobei R₁ wie in Formel I definiert ist,
R₂ und R₃ unabhängig voneinander für Wasserstoff , einen C₁ bis C₄-Alkylrest, einen unsubstituierten oder einen durch unter den Reaktionsbedingungen inerte Gruppen substituierten Phenyl- oder Pyridylrest, oder für einen -COOR₁-Rest stehen, oder einen Rest der Formel (CH₂)ₘ-O-CO-(CH₂)ₚ bedeuten, wobei m eine ganze Zahl von 1 bis 4 und p eine Ganze Zahl von1 bis 6 sein kann,
oder, falls n 1 ist und Q₁ den Rest 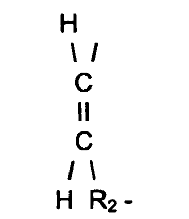 darstellt, stehen R₂ und R₃ zusammen für eine Einfachbindung zwischen zwei benachbarten C-Atomen oder für einen Alkylenrest mit 2 bis 4 C-Atomen falls Y einen o-Phenylenrest oder einen Alkylenrest mit 2 bis 4 C-Atomen und R ein Wasserstoffatom bedeutet,
ansonsten Y dieselbe Bedeutung wie X in Formel I hat, falls n 1 bedeutet, oder falls n für 0 steht, entweder Wasserstoff bedeutet oder zusammen mit R₃ bzw. mit R₃ und der C=C-Doppelbindung, einen gegebenenfalls substituierten, aliphatischen, araliphatischen, aromatischen oder heteroaromatischen Rest mit 1 bis 50 C-Atomen, der durch Sauerstoff, Stickstoff oder Schwefel durchbrochen sein kann, oder Y mit R₃ und der C=C-Doppelbindung einen gegebenenfalls substituierten mono- oder bicyclischen Rest mit 4 bis 20 C-Atomen, der ein oder 2 Heteroatome aus der Gruppe S, N oder O enthalten kann, bedeutet, oder Y und R gemeinsam einen mono- oder bicyclischen Rest mit 4 bis 20 C-Atomen bilden, der ein- oder mehrfach durch unter den Reaktionsbedingungen inerte Gruppen substituiert sein kann
und R wie in Formel I definiert ist, eingesetzt werden.

## Claims

1. Process for the preparation of monocarbonyl or biscarbonyl or hydroxyl compounds by ozonization of unsaturated organic carbon compounds which have one or more olefinic or aromatic double bonds which can be cleaved by ozone in the molecule, and subsequent work-up of the ozonization products, **characterized in that** it comprises reacting unsaturated organic carbon compounds which have one or more olefinic or aromatic double bonds which can be cleaved by ozone in the molecule,
a) in an organic solvent or in aqueous solution in 1 to 2 steps continuously in equipment consisting of two absorption apparatuses, devices for dissipating heat of the reaction and devices for separating the gas and liquid phase, with countercurrent reactant streams, with ozone in stoichiometric amounts or in excess and
b) converting the peroxides which form into the corresponding monocarbonyl or biscarbonyl or hydroxyl compounds either by continuous or discontinuous hydrogenation, oxidation or heating, depending on the reaction parameters from step a),
where, in step a)
the reactant is fed into the first absorption apparatus at a starting concentration which depends on the reactant used and the reaction conditions, whereas the ozone-bearing O₂-stream is introduced into the second absorption apparatus at an ozone concentration which depends on the reactivity of the reactant, such that, in the first absorption apparatus, the reactant used is brought into contact with the ozone stream which is fed into the first absorption apparatus after passing through the second absorption apparatus, as a result of which there is a deficit of ozone in the first absorption apparatus,
then the reaction mixture, following the reaction of the ozone fed into the first absorption apparatus with the correspondingly introduced reactant, emerges from the first absorption apparatus, is separated into a gas phase and a liquid phase, and the liquid phase, which still comprises unreacted reactant, solvent and the corresponding ozonolysis product, is fed into the second absorption apparatus into which the ozone-bearing O₂-stream with the desired starting concentration of ozone is introduced, as a result of which there is an excess of ozone in this apparatus,
and then, when the reaction is complete, the reaction mixture, after emerging from the second absorption column, is again separated into a gas phase and a liquid phase, and then the liquid phase, which now comprises only the corresponding ozonolysis product in the solvent used, is passed to work-up stage b), and the small percentage of ozone present in the gas phase is optionally introduced into the first absorption apparatus for the further reaction of newly introduced reactant.

2. Process according to Claim 1, **characterized in that** the amount of ozone is chosen depending on the reactant used such that, for reactive substances, it corresponds to almost stoichiometric ozone consumption up to about 107% of the stoichiometric amount and, for less reactive substances, an ozone consumption of about 107 to 140%, preferably up to 120%, of the stoichiometric amount, based on the reactant.

3. Process according to Claim 1, **characterized in that** the starting concentration of the reactant is between 1 and 3 mol/l, based on the double bonds, depending on the reactant used and the reaction conditions.

4. Process according to Claim 1, **characterized in that** reactants which react relatively rapidly relative to the solvent or to the ozonolysis product formed are preferably used.

5. Process according to Claim 1, **characterized in that**
the reactant stream is introduced into the first absorption apparatus and the ozone-bearing O₂₋stream is also introduced into the first absorption apparatus, some of the stream for the second apparatus being diverted and fed into said apparatus, such that an ozone split is carried out, as a result of which there is again a deficit of ozone in the first absorption apparatus,
and then, when the reaction in the first absorption apparatus is complete, the reaction mixture is separated into a gas phase and a liquid phase,
the liquid phase, which comprises mainly the corresponding ozonolysis product in the solvent used and residual unreacted reactant, is introduced into the second absorption apparatus,
where it is brought into contact with the diverted ozone stream, as result of which there is an excess of ozone in the second absorption apparatus,
and then, when the reaction is complete, the reaction mixture is again separated into a gas phase and a liquid phase
and the liquid phase, which now comprises only the corresponding ozonolysis product in the solvent used, is passed to the work-up phase (step b).

6. Process according to Claim 5, **characterized in that** the splitting of the ozone-bearing O₂-stream takes place in a ratio of first absorption apparatus to second absorption apparatus of 50 : 50 to 90 : 10, where the O₂-stream fed into absorption apparatus 1 and 2 comprises 4-10% of ozone.

7. Process according to Claim 5 and 6, **characterized in that** the reactant concentration after leaving the first absorption apparatus depends on the splitting ratio of the ozone stream and, in the case of a 90:10 split, is preferably 0.1 mol/l to 0.5 mol/l and, in the case of a 50:50 split, is preferably 0.9 to 2 mol/l.

8. Process according to Claim 1-7, **characterized in that** the absorption apparatus used are apparatuses which effect gas-liquid exchange.

9. Process according to Claim 8, **characterized in that** the absorption apparatuses used are absorption columns, bubble columns, stirred reactors, stirred-tank reactors, mixers or loop reactors.

10. Process according to Claim 1-9, **characterized in that** bubble columns are used as absorption apparatuses for the ozonolysis in the aqueous system.

11. Process according to Claim 10, **characterized in that**, in the case of ozonolysis in the aqueous system, bubble columns are used as absorption apparatuses and an ozone split is carried out according to claim 6.

12. Process according to Claim 1, **characterized in that** the peroxides obtained in step a) are converted into the corresponding monocarbonyl or biscarbonyl or hydroxyl compounds in step b) by continuous or discontinuous hydrogenation, in which the peroxide-containing ozonolysis product solution obtained from the ozonolysis stage, and a hydrogen stream is introduced into a hydrogenation apparatus which ensures an adequate mass transfer of hydrogen into the liquid phase and which has an initial charge of a solvent and a hydrogenation catalyst, optionally with simultaneous metered addition of a basic additive for regulating the pH, and the volume of the reactor solution is kept constant by level-controlled discharge via the filtration unit, as a result of which the peroxide content of the discharged solution, which is below 0.01 mol/l is continuously controlled.

13. Process according to Claim 12, **characterized in that** the hydrogenation apparatuses used are stirred-tank reactors, loop reactors, stirred or unstirred bubble columns or fixed-bed reactors.

14. Process according to Claim 1-13, **characterized in that** monocarbonyl or biscarbonyl or hydroxyl compounds of the general formula I 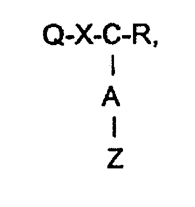 in which
Z is either OH or O and A, when Z is OH, is a single bond and, when Z is O, is a double bond
Q is hydrogen or the radicals 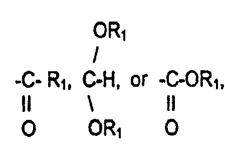 or -OR₁
where R₁ is H or an ester moiety derived from chiral or nonchiral primary, secondary or tertiary alcohols,
X is a straight-chain or branched mono- or divalant, aliphatic alkyl or alkylene radical having 1 to 50 carbon atoms, where this alkyl or alkylene radical may be substituted by one or more groups which are inert under the reaction conditions; an optionally substituted, straight-chain or branched aliphatic alkyl or alkenyl radical having 2 to 50 carbon atoms, where one or more of the -CH₂ groups of the alkyl or alkylene chain is replaced by an oxygen atom, a nitrogen atom, a sulphur atom or an -SO₂ group; a radical of the formula - (CH₂)ₘ-O-CO- (CH₂)ₚ, where m may be an integer from 1 to 4 and p may be an integer from 1 to 6; a phenyl or phenylene radical, where this phenyl or phenylene radical may be substituted by one or more groups which are inert under the reaction conditions; a mono- or divalent alkylarylene or alkylene-arylene radical having 7 to 50 carbon atoms, where these radicals may be substituted by one or more groups which are inert under the reaction conditions; an optionally substituted heterocycle with one or two heteroatoms in the ring or a single bond between two adjacent carbon atoms, and
R is hydrogen, a C₁ to C₂₀-alkyl radical, -OR₁ or the radical 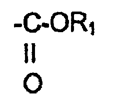 or X and R together form a mono- or bicyclic radical having 4 to 20 carbon atoms which may be mono- or polysubstituted by groups which are inert under the reaction conditions,
are prepared.

15. Process according to Claim 1-14, **characterized in that** the unsaturated organic carbon compounds which have one or more olefinic or aromatic double bonds which can be cleaved by ozone in the molecule used are
compounds of the general formula II 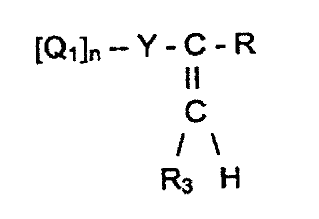 in which n is 0 or 1, Q₁ is hydrogen or the radicals 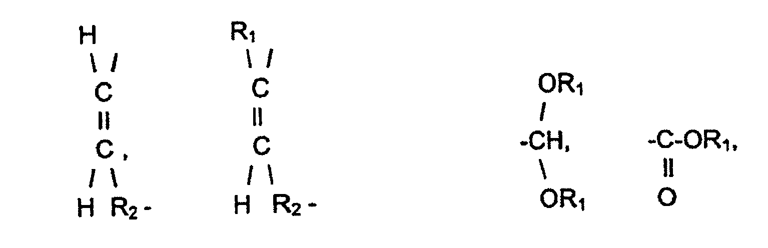 where R₁ is as defined in formula I,
R₂ and R₃, independently of one another, are hydrogen, a C₁ to C₄-alkyl radical, a phenyl or pyridyl radical which is unsubstituted or substituted by groups which are inert under the reaction conditions, or are a -COOR₁ radical, or are a radical of the formula (CH₂)ₘ-O-CO- (CH₂)ₚ,
where m may be an integer from 1 to 4 and p may be an integer from 1 to 6,
or, if n is 1 and Q₁ is the radical 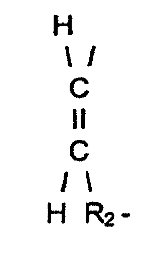 R₂ and R₃ are together a single bond between two adjacent carbon atoms or are an alkylene radical having 2 to 4 carbon atoms if Y is an o-phenylene radical or an alkylene radical having 2 to 4 carbon atoms and R is a hydrogen atom,
otherwise Y has the same meaning as X in formula I, if n is 1, or if n is 0, is either hydrogen or, together with R₃ or with R₃ and the C=C double bond, is an optionally substituted, aliphatic, araliphatic, aromatic or heteroaromatic radical having 1 to 50 carbon atoms which may be interrupted by oxygen, nitrogen or sulphur, or Y with R₃ and the C=C double bond is an optionally substituted mono- or bicyclic radical having 4 to 20 carbon atoms which can contain 1 or 2 heteroatoms from the group S, N or O, or Y and R together form a mono- or bicyclic radical having 4 to 20 carbon atoms which can be mono- or polysubstituted by groups which are inert under the reaction conditions
and R is as defined in formula I.

## Revendications

1. Procédé pour la préparation de composés hydroxylés ou mono- ou biscarbonyle, par ozonisation de composés carbonés organiques insaturés qui comportent dans la molécule une ou plusieurs doubles liaisons oléfiniques ou aromatiques susceptibles d'être coupées par l'ozone, et traitement final subséquent des produits d'ozonisation, **caractérisé en ce que**
a) on fait réagir avec de l'ozone en quantités stoechiométriques ou en excès des composés carbonés organiques insaturés qui comportent dans la molécule une ou plusieurs doubles liaisons oléfiniques ou aromatiques susceptibles d'être coupées par l'ozone, dans un solvant organique ou en solution aqueuse, en 1 ou 2 étapes en continu dans un dispositif consistant en deux appareils d'absorption, en dispositifs pour l'élimination de la chaleur de réaction et en dispositifs pour la séparation de la phase gazeuse et de la phase liquide, à courants en sens opposés de produits de départ et
b) les peroxydes ainsi formés sont convertis en les composés hydroxylés ou mono- ou biscarbonyle correspondants, en fonction des paramètres réactionnels à partir de l'étape a) par hydrogénation continue ou discontinue, oxydation ou chauffage,
dans l'étape a)
le produit de départ étant introduit dans le premier appareil d'absorption à une concentration initiale dépendant du produit de départ utilisé et des conditions réactionnelles, le courant d'O₂ amenant l'ozone étant par contre introduit dans le second appareil d'absorption à une concentration d'ozone dépendant de la réactivité du produit de départ, de sorte que dans le premier appareil d'absorption le produit de départ utilisé est mis en contact avec le courant d'ozone qui est introduit dans le premier appareil d'absorption après avoir traversé le second appareil d'absorption, de sorte qu'une insuffisance d'ozone est présente dans le premier appareil d'absorption, le mélange réactionnel, après la réaction de l'ozone introduit dans le premier appareil d'absorption avec le produit de départ correspondant introduit, sortant ensuite du premier appareil d'absorption, étant séparé en une phase gazeuse et une phase liquide, à la suite de quoi la phase liquide qui contient encore du produit de départ n'ayant pas réagi, du solvant et le produit d'ozonolyse correspondant, est introduite dans le second appareil d'absorption, dans lequel est introduit le courant d'O₂ amenant l'ozone, à la teneur initiale désirée en ozone, de sorte qu'un excès d'ozone est présent dans cet appareil, à la suite de quoi, une fois la réaction terminée, le mélange réactionnel après sa sortie de la deuxième colonne d'absorption est de nouveau séparé en une phase gazeuse et une phase liquide, à la suite de quoi la phase liquide ne contient désormais plus que le produit d'ozonolyse correspondant dans le solvant utilisé, puis on effectue l'étape de traitement final b) et le faible pourcentage d'ozone contenu dans la phase gazeuse est éventuellement introduit dans le premier appareil d'absorption, pour la mise en réaction ultérieure de produit de départ nouvellement introduit.

2. Procédé selon la revendication 1, **caractérisé en ce que** la quantité d'ozone est choisie en fonction du produit de départ utilisé, de manière à ce qu'elle corresponde pour des substances réactives à une consommation d'ozone presque stoechiométrique jusqu'à environ 107 % de la quantité stcechiométrique et pour des substances peu réactives à une consommation d'ozone d'environ 107 à 140 %, de préférence 120 %, de la quantité stoechiométrique, par rapport au produit de départ.

3. Procédé selon la revendication 1, **caractérisé en ce que** la concentration initiale du produit de départ est comprise, en fonction du produit de départ utilisé et des conditions réactionnelles, entre 1 et 3 moles/l, par rapport aux doubles liaisons.

4. Procédé selon la revendication 1, **caractérisé en ce qu'**on utilise des produits de départ réagissant rapidement par rapport au solvant ou au produit d'ozonolyse formé.

5. Procédé selon la revendication 1, **caractérisé en ce que**
le produit de départ est envoyé dans le premier appareil d'absorption et le courant d'O₂ amenant l'ozone est également introduit dans le premier appareil d'absorption, une partie du courant étant dérivée pour le second appareil et introduite dans celui-ci, de sorte qu'un partage de l'ozone est effectué, de manière qu'une insuffisance d'ozone soit de nouveau présente dans le premier appareil d'absorption,
à la suite de quoi, une fois la réaction terminée dans le premier appareil d'absorption, le mélange réactionnel est scindé en une phase gazeuse et une phase liquide,
la phase liquide, qui contient principalement le produit d'ozonolyse correspondant dans le solvant utilisé et le produit de départ résiduel n'ayant pas réagi, est introduite dans le second appareil d'absorption, où elle est mise en contact avec le courant d'ozone dérivé, de sorte qu'un excès d'ozone est présent dans le second appareil d'absorption,
à la suite de quoi, une fois la réaction terminée, le mélange réactionnel est de nouveau scindé en une phase gazeuse et une phase liquide,
et la phase liquide, qui désormais ne contient plus que le produit d'ozonolyse correspondant dans le solvant utilisé, est envoyée à la phase de traitement final (étape b).

6. Procédé selon la revendication 5, **caractérisé en ce que** la division du courant d'O₂ amenant l'ozone s'effectue en un rapport du premier appareil d'absorption au second appareil d'absorption allant de 50:50 à 90:10, le courant d'O₂ introduit dans l'appareil d'absorption 1 et l'appareil d'absorption 2 contenant 4-10 % d'ozone.

7. Procédé selon la revendication 5 ou 6, **caractérisé en ce que** la concentration du produit de départ à la sortie du premier appareil d'absorption dépend du rapport de partage du courant d'ozone et, à un partage de 90:10 va de préférence de 0,1 mole/l à 0,5 mole/l et à un partage de 50:50 va de préférence de 0,9 à 2 moles/l.

8. Procédé selon l'une quelconque des revendications 1 à 7, **caractérisé en ce qu'**on utilise comme appareil d'absorption des appareils qui réalisent un échange gaz-liquide.

9. Procédé selon la revendication 8, **caractérisé en ce qu'**on utilise comme appareils d'absorption des colonnes d'absorption, des colonnes à barbotage, des réacteurs à agitation, des cuves à agitation, des mélangeurs ou des réacteurs à boucle.

10. Procédé selon l'une quelconque des revendications 1 à 9, **caractérisé en ce que** pour l'ozonolyse en système aqueux on utilise comme appareils d'absorption des colonnes à barbotage.

11. Procédé selon la revendication 10, **caractérisé en ce que** dans l'ozonolyse en système aqueux on utilise comme appareils d'absorption des colonnes à barbotage et on effectue un partage d'ozone selon la revendication 6.

12. Procédé selon la revendication 1, **caractérisé en ce que** les peroxydes obtenus dans l'étape a) sont convertis dans l'étape b) en composés hydroxylés
ou mono- ou biscarbonyle correspondants par hydrogénation continue ou discontinue, dans lequel la solution de produit d'ozonolyse contenant des peroxydes, obtenue à partir de l'étape d'ozonolyse, et un courant d'hydrogène sont introduits dans un appareil d'hydrogénation, assurant un transfert suffisant de masse d'hydrogène dans la phase liquide, dans lequel sont disposés au préalable un solvant et un catalyseur d'hydrogénation, éventuellement avec addition dosée simultanée d'un additif basique pour la régulation du pH, à la suite de quoi le volume de la solution réactionnelle est maintenu constant par décharge constante sur l'unité de filtration, de sorte que la teneur en peroxydes de la solution déchargée, qui est inférieure à 0,01 mole/l, est réglée en continu.

13. Procédé selon la revendication 12, **caractérisé en ce qu'**on utilise comme appareils d'hydrogénation des cuves à agitation, des réacteurs à boucle, des colonnes à barbotage et munies ou non d'agitation, ou des réacteurs à lit fixe.

14. Procédé selon l'une quelconque des revendications 1 à 13, **caractérisé en ce qu'**on prépare des composés hydroxylés ou mono- ou biscarbonyle de formule générale I 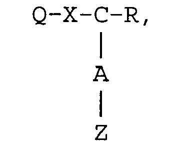 dans laquelle
Z représente soit OH, soit 0, et A représente, lorsque Z est OH, une liaison simple, et lorsque Z est O, une double liaison
Q représente un atome d'hydrogène ou les radicaux 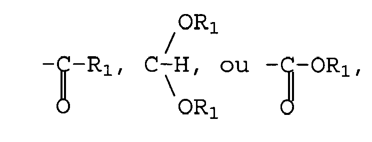 ou -OR₁
R₁ représentant H ou un fragment ester qui dérive d'alcools primaires, secondaires ou tertiaires, chiraux ou non chiraux,
X représente un radical alkyle ou alkylène aliphatique mono- ou divalent à chaîne droite ou ramifiée, ayant de 1 à 50 atomes de carbone, ce radical alkyle ou alkylène pouvant être substitué par un ou plusieurs groupes qui sont inertes dans les conditions réactionnelles ; un radical alkyle ou alkylène aliphatique à chaîne droite ou ramifiée, éventuellement substitué, ayant de 2 à 50 atomes de carbone, un ou plusieurs des groupes CH₂ de la chaîne alkyle ou alkylène pouvant être remplacé(s) par un atome d'oxygène, un atome d'azote, un atome de soufre ou un groupe -SO₂ ; un radical de formule - (CH₂)ₘ-O-CO- (CH₂)ₚ, m étant un nombre entier pouvant aller de 1 à 4 et p étant un nombre entier pouvant aller de 1 à 6 ; un radical phényle ou phénylène, ce radical phényle ou phénylène pouvant être substitué par un ou plusieurs groupes qui sont inertes dans les conditions réactionnelles ; un radical alkylarylène ou alkylène-arylène ayant de 7 à 50 atomes de carbone, ces radicaux pouvant être substitués par un ou plusieurs groupes qui sont inertes dans les conditions réactionnelles ; un hétérocycle éventuellement substitué comportant un ou deux hétéroatomes dans le cycle ou une simple liaison entre deux atomes de carbone contigus, et
R représente un atome d'hydrogène, un radical alkyle en C₁-C₂₀, -OR₁ ou le
radical 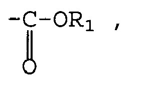 ou bien X et R forment ensemble un radical mono- ou bicyclique ayant de 4 à 20 atomes de carbone, qui peut être une ou plusieurs fois substitué par des groupes inertes dans les conditions réactionnelles.

15. Procédé selon l'une quelconque des revendications 1 à 14, **caractérisé en ce qu'**en tant que composés carbonés organiques insaturés qui comportent dans la molécule une ou plusieurs doubles liaisons oléfiniques ou aromatiques susceptibles d'être coupées par l'ozone
on utilise un composé de formule générale II 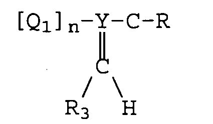 dans laquelle n est 0 ou 1, Q₁ représente un atome d'hydrogène ou les radicaux 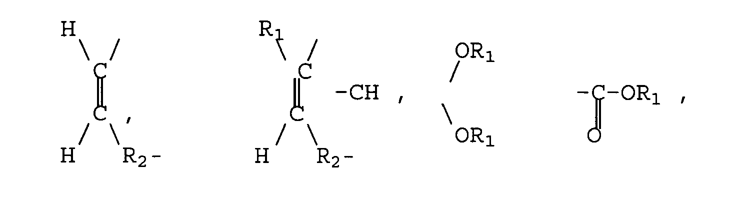 R₁ étant tel que défini dans la formule I,
R₂ et R₃ représentent, indépendamment l'un de l'autre, un atome d'hydrogène, un radical alkyle en C₁-C₄, un radical phényle ou pyridyle non substitué ou substitué par des groupes inertes dans les conditions réactionnelles, ou un radical -COOR₁, ou un radical de formule -(CH₂)ₘ-O-CO-(CH₂)ₚ, m étant un nombre entier pouvant aller de 1 à 4 et p étant un nombre entier pouvant aller de 1 à 6,
ou bien, lorsque n est 1 et Q₁ représente le radical 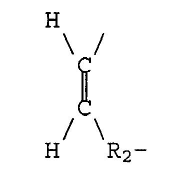 R₂ et R₃ représentent ensemble une simple liaison entre deux atomes de carbone contigus ou représentent un radical alkylène ayant de 2 à 4 atomes de carbone lorsque Y représente un radical o-phénylène ou un radical alkylène ayant de 2 à 4 atomes de carbone et R représente un atome d'hydrogène,
sinon Y a la même signification que X dans la formule I, lorsque n représente 1, ou si n représente 0, soit représente un atome d'hydrogène, soit forme conjointement avec R₃ ou avec R₃ et la double liaison C=C un radical aliphatique, araliphatique, aromatique ou hétéroaromatique éventuellement substitué ayant de 1 à 50 atomes de carbone, qui peut être interrompu par un atome d'oxygène, d'azote ou de soufre, ou bien Y représente avec R₃ et la double liaison C=C un radical mono- ou bicyclique éventuellement substitué ayant de 4 à 20 atomes de carbone, qui peut comporter un ou 2 hétéroatomes choisis parmi S, N et O, ou Y et R forment ensemble un radical mono- ou bicyclique ayant de 4 à 20 atomes de carbone, qui peut être une ou plusieurs fois substitué par des groupes inertes dans les conditions réactionnelles
et R est tel que défini dans la formule I.
